(19) Europäisches Patentamt
European Patent Office
Office européen des brevets

(11) Publication number: **0 013 790**
**B1**

# (12) EUROPEAN PATENT SPECIFICATION

(45) Date of publication of patent specification: **22.09.82**

(51) Int. Cl.[3]: **C 07 D 498/04, A 61 K 31/42**

(21) Application number: **79301283.2**

(22) Date of filing: **02.07.79**

## (54) Clavulanic acid derivatives, their preparation and pharmaceutical compositions containing them.

(30) Priority: **05.07.78 GB 2890678**

(43) Date of publication of application:
**06.08.80 Bulletin 80/16**

(45) Publication of the grant of the patent:
**22.09.82 Bulletin 82/38**

(84) Designated Contracting States:
**AT BE CH DE FR GB IT LU NL SE**

(56) References cited:
**DE - A - 2 646 004**

The file contains technical information submitted after the application was filed and not included in this specification

(73) Proprietor: **BEECHAM GROUP PLC**
**Beecham House Great West Road**
**Brentford Middlesex (GB)**

(72) Inventor: **Gasson, Brian Charles**
**9 Clarence Walk**
**Redhill, Surrey (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

**EP 0 013 790 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Clavulanic acid derivatives, their preparation and pharmaceutical compositions containing them

The present invention relates to certain ethers of clavulanic acid, to a process for their preparation and to pharmaceutical compositions containing them.

Belgian Patent No. 847045 discloses that ethers of clavulanic acid have $\beta$-lactamase inhibitory and antibacterial activity. A group of hitherto unsuggested ethers have now been prepared that possess $\beta$-lactamase inhibitory and antibacterial activity.

The present invention provides the compounds of the formula (I):

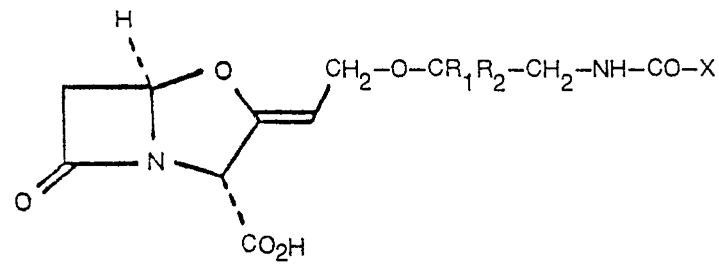

(I)

and salts and esters thereof wherein $R_1$ is a hydrogen atom or methyl group; $R_2$ is a hydrogen atom or methyl group; and X is a group of the sub-formula (a), (b), (c) or (d):

(a) $—CHR_3R_4$ (b) $—CH_2—CHR_3R_4$ (c) $—CH_2—CH_2—CHR_3R_4$ (d) 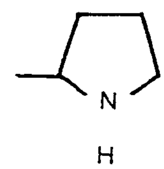

wherein $R_3$ is a hydrogen atom or a lower alkyl, aryl, aralkyl or substituted alkyl group; and $R_4$ is an amino group or a carboxylic acid group.

When used herein the term "lower alkyl" means a group of up to 4 carbon atoms and more suitably of 1 or 2 carbon atoms. When used herein the term "aryl" means a phenyl group or a phenyl group substituted by a fluorine or chlorine atom or a methoxyl, methyl or hydroxyl group. When used herein the term "aralkyl" means a lower alkyl group substituted by an aryl group. When used herein the term "substituted alkyl" means a lower alkyl group substituted by an amino, carboxamide or carboxylic acid group.

Favourably $R_1$ is a hydrogen atom. Favourably $R_2$ is a hydrogen atom.

A particularly apt group X is that of the sub-formula (a) so that a particularly favoured sub-group of compounds of the formula (I) are those of the formula (II):

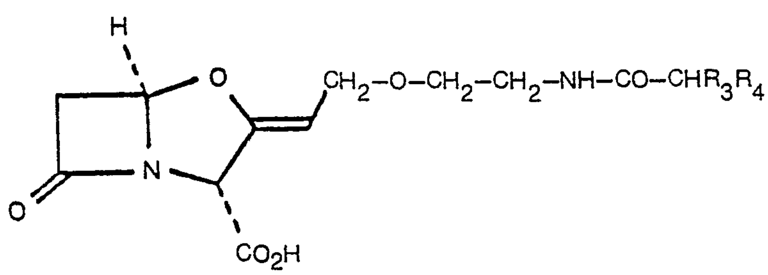

(II)

and salts and ester thereof wherein $R_3$ and $R_4$ are as defined in relation to formula (I).

An especially suitable group of compounds of the formula (I) is that of the formula (III):

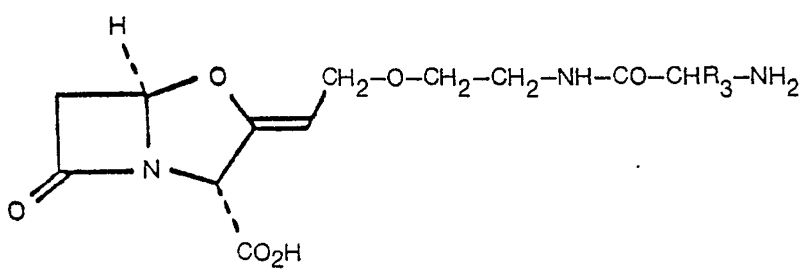

(III)

wherein $R_3$ is as defined in relation to formula (I).

A further especially suitable group of compounds of the formula (I) is that of the formula (IV):

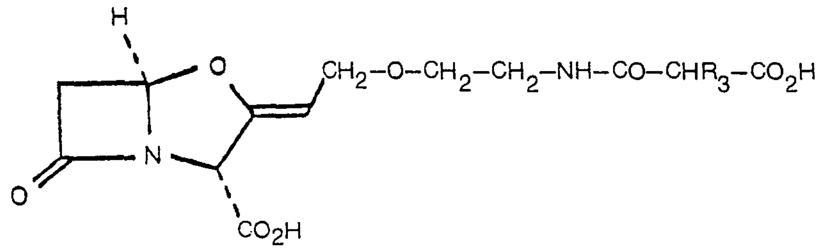

(IV)

and salts and esters thereof wherein $R_3$ is as defined in relation to formula (I).

Suitable values for $R_3$ in the compounds of the formulae (I), (II), (III) and (IV) include the hydrogen atom and the methyl, ethyl, n-propyl, isopropyl, phenyl, p-hydroxyphenyl, p-chlorophenyl, p-methoxyphenyl, benzyl, carboxymethyl and 5-$(CH_2)_3$—$CH_2NH_2$ group.

A favoured value for $A_3$ is the hydrogen atom.

A further favoured value for $R_3$ is the methyl group.

The compounds of the formula (I) may be presented in the form of a salt if desired. In general these compounds of the formula (I) wherein $R_4$ is an amino group are preferred in the form of the zwitterion but esters of these compounds may be in the form on an acid addition salt, for example a salt with a pharmaceutically acceptable organic or inorganic acid. Suitable acids for this purpose include hydrochloric, orthophosphoric, sulphuric, methanesulphonic, toluenesulphonic, acetic, propionic, citric, lactic, malic, succinic, salicylic or the like acid. In general these compounds of the formula (I) wherein $R_4$ is a carboxylic acid group are presented in the form of a mono-salt or more suitably a di-basic salt. Suitable cations for use in such salts include the sodium, potassium, calcium, magnesium, ammonium and the like. The lithium salts may also be employed. Mono-esters of the di-acids within formula (I) may also be salted, for example by one of the aforementioned cations.

The compounds of the formula (I) may be presented in the form of an ester. Most suitably such esters are cleavable to yield the parent acid or its salt in-vivo or by chemical means such as hydrolysis or hydrogenolysis.

Suitable esters include those of the part formulae (a)—(b):

(a) —CO—O—$A_1$ (b) —CO—O—$CHA_2$—O—$COA_3$

(c) 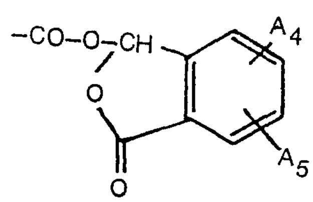 (d) —CO—O—$CHA_6A_7$

wherein $A_1$ is a lower alkyl, lower alkenyl, lower alkynyl, or a lower alkyl group substituted by a lower alkoxyl, lower acyl, $CF_3$, $CCl_3$, $CO.CH_3$, $CO.C_6H_5$ or carboxylic acid group or a salt or lower alkyl ester thereof; $A_2$ is a hydrogen atom or a methyl group; $A_3$ is a lower alkyl, lower alkoxyl, phenyl or benzyl group; $A_4$ is a hydrogen atom or a methyl or methoxyl group; $A_5$ is a hydrogen atom or a methyl or methoxyl group; $A_6$ is a phenyl group or a phenyl group substituted by a fluorine, chlorine or bromine atom or a methyl, methoxyl or nitro group; and $A_7$ is a hydrogen atom or a phenyl group or a phenyl group substituted by a fluorine, chlorine or bromine atom or a methyl, methoxyl or nitro group.

Particularly suitable values for $A_1$ include the methyl, ethyl, ethoxymethyl, 2-methoxyethyl, 2-hydroxyethyl, 2,2,2-trifluoroethyl, 2,2,2-trichloroethyl, propyl, propenyl, propynyl, 2-oxopropyl, benzoylmethyl, carboxymethyl, sodium carboxymethyl, benzyloxycarboxymethyl and the like.

Particularly suitable values for $CHA_2$—$OCOA_3$ include acetoxymethyl, $\alpha$-acetoxyethyl, pivaloyloxymethyl, $\alpha$-pivaloyloxyethyl, ethoxycarbonyloxymethyl, $\alpha$-ethoxycarbonyloxyethyl, benzoyloxymethyl and the like.

Particularly suitable $A_4$ and $A_5$ both represent hydrogen atoms.

Particularly suitable values for $A_6$ include the phenyl, p-chlorophenyl, p-bromophenyl, p-methoxyphenyl, p-nitrophenyl and the like group. Particularly suitably $A_7$ is a hydrogen atom or a phenyl, p-chlorophenyl, p-bromophenyl, p-methoxyphenyl, p-nitrophenyl or the like group. More favourably $A_7$ is a hydrogen atom.

The —$CHR_3R_4$ moiety in the compounds of the formulae (I)—(IV) may be presented as R-isomer or S-isomer or mixtures thereof when $R_3$ is other than a hydrogen atom. When $R_4$ is an amino group the compound may advantageously be one in which the —NH—CO—$CHR_3$—NH moiety is one previously described as suitable as a 6-position substituent in penicillins.

The present invention also provides a process for the preparation of a compound of the formula (I)

or a salt or ester thereof which process comprises the deprotection of a compound of the formula (V):

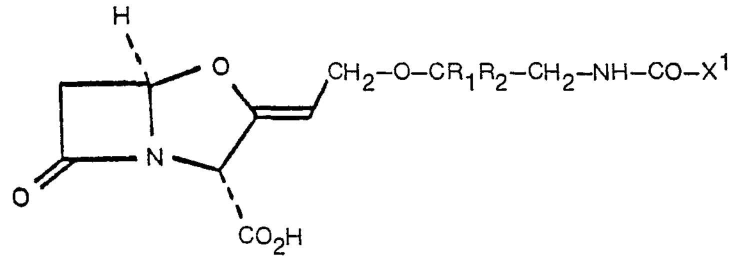

(V)

or a salt or ester thereof wherein $R_1$ and $R_2$ are as defined in relation to formula (I) and $X^1$ is a group of the sub-formula (a), (b), (c) or (d) in which the amino or carboxylic group is protected.

Apt protected amino and protected carboxylic acid groups include those known from the chemistry of peptides, penicillins or cephalosporins.

Particularly suitable protected groups $X^1$ include those of the sub-formulae (e) and (f):

(e) —NH—CO—$OR_5$ (f) —CO—$OR_5$

wherein $R_5$ is a benzyl or substituted benzyl group.

Favourably $R_5$ is an unsubstituted benzyl group. Suitable substituted benzyl groups include chlorobenzyl, bromobenzyl, methoxybenzyl, nitrobenzyl, methylbenzyl, benzhydryl or other like groups.

The compounds of the formula (V) or a salt or ester thereof wherein $X^1$ is a group of the sub-formulae (e) or (f) may be converted to a corresponding compound of the formula (I) or its salt or ester by catalytic hydrogenation.

Such hydrogenation reactions may be carried out using a lower, medium or high pressure of hydrogen and it is often convenient to employ an atmospheric or slightly superatmospheric pressure of hydrogen. The catalyst employed is normally a noble metal catalyst and is preferably a palladium catalyst such as palladium on carbon or the like. Any convenient non-extreme temperature may be used, for example 0° to 40°C but in general it is most conveniently carried out at ambient temperature. The solvent employed may be a conventional solvent used in hydrogenation reactions such as tetrahydrofuran, aqueous tetrahydrofuran, ethanol, ethylacetate or the like.

It is advantageous to carry out the hydrogenation reaction on an ester of the compound of formula (V) which is cleavable by hydrogenation to yield the parent carboxylic acid. Such esters include benzyl and substituted benzyl esters such as the chlorobenzyl, bromobenzyl, methoxybenzyl, nitrobenzyl, methylbenzyl, benzhydryl or the like ester. Particularly suitable esters include the benzyl, p-methoxybenzyl and p-nitrobenzyl esters of which the benzyl is preferred. Hydrogenation of such a hydrogenolysable ester of a compound of the formula (V) wherein $R_4^1$ is a group of the sub-formula (e) leads to the preparation of the desirable zwitterionic form of a compound of the formula (I) wherein $R_4$ is an amino group. Hydrogenation of such a hydrogenolysable ester of a compound of the formula (V) wherein $R_4^1$ is a group of the sub-formula (f) leads to the diacid of a compound of the formula (I) wherein $R_4$ is a carboxylic acid group. The acid may be thereafter converted to a salt by neutralisation with a base such as LiOH, $NaHCO_3$, KOH, $Ca(OH)_2$, $Ba(OH)_2$, $Mg(OH)_2$, $NH_4OH$, $HN(C_2H_5)_3$, $N(C_2H_5)_3.HO_2CCH_3$, Na ethylhexanoate, K ethylhexanoate or the like. Alternatively the hydrogenation may be performed in the presence of a base such as $NaHCO_3$, $Na_2CO_3$, $K_2CO_3$, $CaCO_3$, $MgCO_3$, $LiHCO_3$, $N(C_2H_5)_3.HO_2CCH_3$ or the like under which conditions the desired salt is obtained directly. The lithium salts of the diacids within formula (I) tend to be more easily prepared in pure crystalline form than other salts of the diacids within formula (I). It is therefore often convenient to first form the lithium salt and then convert this into an alternative salt by ion-exchange, for example by passing a solution of the lithium salt through a bed of cation exchange resin in sodium, potassium, calcium, ammonium or like form. Suitable cation exchange resins include Amberlite IR120 and equivalent resins.

If the hydrogenation reaction is performed on a non-hydrogenolysable ester of the compound of the formula (V) then naturally an ester of the compound of the formula (I) results. Such esters of compounds wherein $R_4$ is an amino group may be salted by an organic or inorganic acid present during hydrogenation or less favourably added thereafter. Such acids are normally pharmaceutically acceptable such as hydrochloric, acetic, citric, malic or the like. Esters of the compounds of the formula (I) wherein $R_4$ is a carboxyl group may be salted as previously described. The monoesters of the compounds wherein $R_4^1$ is a carboxyl group or a salt thereof may often be hydrolysed to yield the corresponding salt, for example by mild base hydrolysis. Suitable esters for this include methyl, methoxymethyl or the like esters. Suitably these esters may be hydrolysed by maintaining the pH of the medium at 7.5 to 9 until hydrolysis is effected. The pH may be maintained in this desired range in a pH-stat by the addition of a solution or suspension of a base such as LiOH, NaOH, KOH, $Ca(OH)_2$, $Mg(OH)_2$, $NaHCO_3$, $NaCO_3$, $KHCO_3$ or the like at a rate which prevents the accumulation of excess base which would cause the pH to increase unacceptably.

The useful intermediates of the formula (V) and their salts and esters form an aspect of this invention.

The compounds of the formula (V) and salts and esters thereof may be prepared by the reaction of a compound of the formula (VI):

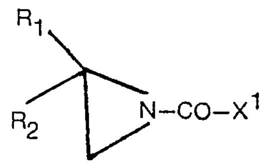

(VI)

wherein $R_1$, $R_2$, $R_3$ and $X^1$ are as defined in relation to formula (V) with an ester of clavulanic acid and thereafter de-esterifying the resulting ester of the compound of the formula (V) if desired.

The addition reaction may be performed in an aprotic medium such as dichloromethane, ethyl acetate or the like at a depressed temperature such as −90° to 0°C and most aptly initially at −80° to −40°C and thereafter slowly allowing the reaction mixture to warm to ambient temperature.

The ester of the compound of the formula (V) may be de-esterified by the processes hereinbefore described.

The compounds of the formula (VI) may be prepared by the acylation of aziridine or a substituted aziridine in conventional manner.

The present invention also provides pharmaceutical composition which comprise a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics. Such compositions may be formulated in known manner, for example by mixing.

Injectable or infusable compositions of salts of a compound of the formula (I) are particularly suitable as high blood levels of a compound of the formula (I) can occur after administration by injection or infusion. Thus, one preferred composition aspect of this invention comprises an injectable salt of a compound of the formula (I) in sterile form, for example the sterile sodium or potassium salt. Most suitably the composition is in unit dosage form.

Unit dose compositions comprising a compound of the formula (I) or a salt or ester thereof adapted for oral administration form a further preferred composition aspect of this invention.

The compound of the formula (I) or its salt or ester may be present in the composition as sole therapeutic agent or it may be present together with other therapeutic agents such as a $\beta$-lactam antibiotic. Suitable $\beta$-lactam antibiotics for inclusion in the compositions of this invention include benzylpenicillin, phenoxymethylpenicillin, carbenicillin, azidocillin, propicillin, ampicillin, amoxycillin, epicillin, ticarcillin, cyclacillin, cefatriazine, pirbenicillin, $\alpha$-sulphonyloxybenzylpenicillin, cephaloridine, cephalothin, cefazolin, cephalexin, cephacetril, cephamandole nafate, cephapirin, cephradine, 4-hydroxycephalexin, cefaparole, cephaloglycine, and other well known penicillins and cephalosporins or pro-drugs therefore such as hetacillin, metampicillin, 4-acetoxyampicillin, the acetoxymethyl, $\alpha$-ethoxycarbonyloxyethyl, pivaloyloxymethyl or phthalidyl esters of ampicillin or amoxycillin or the phenyl, tolyl or indanyl ester of carbenicillin or ticarcillin or the like. Such compounds are frequently used in the form of a hydrate and/or salt such as a sodium or potassium salt of a carboxyl group, or hydrochloride or amine functions and the like.

Naturally if the penicillin or cephalosporin present in the composition is not suitable for oral administration then the composition will be adapted for perenteral administration.

When present together with a cephalosporin or penicillin, the ratio of a compound of the formula (I) or its salt or ester present to the other antibacterial agent may vary over a wide range of ratios, for example 3:1 to 1:10 and advantageously may be from 1:1 to 1:8, for example , 1:2, 1:3, 1:4, 1:5 or 1:6.

The total quantity of compound of the formula (I) in any unit dosage form will normally be between 25 and 1000 mg and will usually be between 50 and 500 mg, for example about 62.5, 100, 125, 150, 200 or 250 mg.

Compositions of this invention may be used for the treatment of infections of inter alia, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example as 1—6 doses, more usually 2—4 doses.

The penicillin or cephalosporin in synergistic compositions of this invention will normally be present up to or at approximately the amount at which it is conventionally used.

Particularly favoured compositions of this invention will contain from 150—1000 mg of amoxycillin, ampicillin or a pro-drug therefore and from 25—500 mg of a compound of the formula (I) or a salt or ester thereof and more suitably from 200—750 mg of amoxycillin or a salt thereof and from 50—250 mg of a salt of a compound of the formula (I).

Most suitably this form of the composition will comprise ampicillin or its salt or amoxycillin or its salt.

The materials present in such compositions may be hydrated if required for example ampicillin thrihydrate or amoxycillin trihydrate may be employed. The weights of the antibiotics in such compositions are expressed on the basis of antibiotic theoretically available from the composition and not on the basis of the weight of prodrug.

The compositions of this invention may be used to treat infections caused by strains of gram-positive and gram-negative bacteria such as *Staphylococcus aureus, Escherichia coli, Klebsiella aerogenes, Haemophilus influenzae, Neisseria gohorrea, Proteus mirabilis, Proteus vulgaris, Pseudomonas aeruginosa, Bacteroides fragilis* and the like including many $\beta$-lactamase producing strains including those of Serratia marcesceus Branhamella Catarrhalis.

The compositions of this invention may be administered so that the therapeutic effect is achieved without any clear signs of acute toxicity being seen.

### Example 1

Benzyl 9-O-[2-(2-benzyloxycarbonyl)acetamido]ethylclavulanate

A solution of benzyl clavulanate (1.445 gm) and N-(2-benzyloxycarbonyl) acetyl aziridine (1.1 gm) in dry methylene chloride (25 ml) was cooled in a dry-ice/acetone bath and treated with boron trifluoride etherate (0.1 ml). After 0.5 hours the mixture was allowed to warm to room temperature over 2 hours. Excess sodium bicarbonate solution (1 N) was then added and the organic phase separated. The aqueous phase was extracted with chloroform (2 × 10 ml) and the combined organic phases were then washed with brine (20 ml) dried over magnesium sulphate and evaporated. This initial product was purified by column chromatography (Kieselgel 2:1 going to 1:1 cyclohexane:ethyl acetate) to yield after evaporation benzyl 9-O-[2-(2-benzyloxycarbony)acetamido]ethylclavulanate (1.754 gm), m.pt. 77—80° (from cyclohexane-ethyl acetate).

I.R. $\nu_{max}$(nujol) 3250, 1795, 1740, 1695 and 1645 $cm^{-1}$.
N.M.R. $\delta$ ($CDCl_3$) 3.02 (1H, d, J = 17Hz), 3.32 (2H, s), 3.3—3.55 (5H, m), 4.04 (2H, d, J = 7Hz, 4.78 (1H, broad t, J = 7Hz), 5.08 (1H, broad s), 5.14 (2H, s) 5.18 (2H, s), 5.66 (1H, d, J = 3Hz), 7.14 (1H, broad), 7.34 (10H, s).

Analysis

| | | | |
|---|---|---|---|
| Found: | C, 63.76; | H, 5.45; | N, 5.61. |
| $C_{29}H_{28}N_2O_8$ requires: | C, 63.77; | H, 5.55; | N, 5.51. |

### Example 2

Disodium 9-O-[2-(2-carboxy)acetamido]ethylclavulanate

A solution of benzyl 9-O-[2-(2-benzyloxycarbonyl)acetamido]ethylclavulanate (1 gm) in tetrahydrofuran (30 ml) was hydrogenated over 10% palladium charcoal (300 mgs) for 20 minutes. The solution was filtered through celite and the filter cake washed with tetrahydrofuran (10 ml). The filtrate was diluted with water (20 ml) and treated with a solution of sodium bicarbonate (330 mgs) in water (5 ml). Most of the tetrahydrofuran was removed by evaporation. The aqueous solution was then extracted with ethyl acetate (2 × 5 ml) and freeze dried to yield disodium 9-O-[2-(2-carboxy)acetamido]ethylclavulanate (598 mg).

I.R. $\nu_{max}$ (KBr) 1783, 1610 (v. broad) $cm^{-1}$.
N.M.R. $\delta$ ($D_2O$) 3.06 (1H, d, J = 17Hz), 3.17 (2H, s), 3.25—3.65 (5H, m), 4.11 (2H, d, J = 7Hz), 6.88 (1H, broad, J = 7Hz), 4.94 (1H, s), 5.72 (1H, d, J = 3Hz).

### Example 3

Benzyl 9-O-[N-($N^1$-benzyloxycarbonyl-L-alanyl)-2-aminoethyl]clavulanate

A solution of benzyl clavulanate (1.734 gm) and 1-(N-benzyloxycarbonyl)-L-alanyl aziridine (1.49 gm) in dry methylene chloride (30 ml) was cooled in a dry-ice/acetone bath and treated with boron trifluoride etherate (0.12 ml). The solution was allowed to warm to room temperature over 2.5 hours and the excess sodium bicarbonate solution (1 N) was added. The organic phase was separated and the aqueous phase extracted with chloroform (2 × 15 ml). The combined organic phases were washed with brine (20 ml), dried over magnesium sulphate and evaporated. The product was purified by column chromatography using gradient elution (Kieselgel 1:1 ethyl acetate:cyclohexane going to neat ethyl acetate) to yield benzyl 9-O-[N-($N^1$-benzyloxycarbonyl-L-alanyl)-2-aminoethyl]clavulanate (2.755 g), m.p. 72—74 (from ethyl acetate-cyclohexane).

I.R. $\nu_{max}$(nujol) 3250, 1800, 1740, 1695, 1650 $cm^{-1}$.
N.M.R. $\delta$ ($CDCl_3$) 1.33 (3H, d, J = 7Hz), 2.99 (1H, d, J = 17Hz), 3.25—3.55 (5H, m), 4.01 (2H, d, J = 7Hz), 4.18 (1H, quintet, J = 7Hz), 4.74 (1H, broad t, J = 7Hz), 5.06 (3H, s), 5.16 (2H, s), 5.44 (1H, broad d, J = 7Hz), 5.64 (1H, d, J = 3Hz), 6.33 (1H broad, 7.30 (10H, s).

Analysis
$C_{28}H_{31}N_3O_8$ requires: C, 62.56; H, 5.81; N, 7.82.
Found: C, 62.62; H, 5.47; N, 7.74.

Example 4

9-O-[N-L-alanyl)-2-aminoethylclavulanic acid

A solution of benzyl 9-O-[N-(N¹-benzyloxycarbonyl-L-alanyl)-2-aminoethyl]clavulanate (1.0 gm) in aqueous tetrahydrofuran (10 ml water, 20 ml tetrahydrofuran) was hydrogenated over 10% palladium charcoal (300 mgs) for 0.5 hours. The mixture was filtered through celite and the filter cake washed with water (10 ml). The combined filtrates were evaporated to remove most of the tetrahydrofuran and the remaining aqueous solution extracted with ethyl acetate (3 x 15 ml). The solution was filtered through celite and evaporated below 30° to about 1—2 ml. Ethanol (3 ml) was added dropwise to the solution and the solution scratched to induce crystallisation. More ethanol was added in small portions to complete crystallisation. The crystals were filtered off and dried to yield 9-O-(N-L-alanyl)-2-aminoethylclavulanic acid (337 mg).

I.R. $\nu_{max}$ (KBr) 1780, 1689, 1625 $cm^{-1}$.
N.M.R. $\delta$ ($D_2O$), 1.41 (3H, d, J = 7Hz), 3.00 (1H, d, J = 17Hz), 3.2—3.65 (5H, m) 3.96 (1H, q. J = 7Hz), 4.05 (2H, d, J = 7Hz), 4.79 (1H, broad t, J = 7Hz), 4.87 (1H, s), 5.65 (1H, d, J = 3Hz).

Analysis
$C_{13}H_{19}N_3O_6$ requires: C, 49.84; H, 6.11; N, 13.41.
Found: C, 49.89; H, 6.38; N, 13.30.

Example 5

Benzyl 9-O-[N-(N-benzyloxycarbonylglycyl)-2-aminoethyl]clavulanate

A solution of benzyl clavulanate (289 mg) and 1-(N-benzyloxycarbonyl)glycyl aziridine (230 mgs) in dry methylene chloride (5 ml) was cooled in a dry-ice/acetone bath and treated with boron trifluoride etherate (0.02 ml). After ½ hr the mixture was allowed to warm to room temperature for 1½ hours. Excess sodium bicarbonate solution was then added and the organic phase separated. The aqeuous phase was extracted twice with chloroform and the combined organic phases were washed with brine, dried over magnesium sulphate an evaporated. The product was isolated by column chromatography using gradient elution (Kieselgel, 1:1 ethyl acete:cyclohexane going to neat ethyl acetate to yield benzyl 9-O-[N-(N-benzyloxycarbonylglycyl)-2-aminoethyl]clavulanate (302 mgs).

I.R. $\nu_{max}$ (film) 3330, 1805, 1730 and 1680 $cm^{-1}$.
N.M.R. $\delta$ ($CDCl_3$) 2.99 (1H, d, J = 17Hz), 3.3—3.55 (5H, m), 3.79 (2H, d, J = 6Hz), 4.01 (2H, d, J = 7Hz), 4.75 (1H, broad t, J = 7Hz), 5.06 (1H, s), 5.09 (2H, s), 5.17 (2H, s), 5.57 (1H, broad t, J = 6Hz), 5.64 (1H, d, J = 3Hz), 6.35 (1H, broad s), 7.32 (10H, s).

Example 6

9-O-(N-Glycyl)-2-aminoethylclavulanic acid

A solution of benzyl 9-O-[N-(N¹-benzyloxycarbonylglycyl)-2-aminoethyl]clavulanate (1.0 gm) in tetrahydrofuran (20 ml) and water (10 ml) was hydrogenated over 10% palladium charcoal (300 mgs) for ½ hour. The mixture was filtered through celite and the filter cake washed with water. The filtrate was evaporated to remove most of the tetrahydrofuran, and the remaining aqueous solution was extracted three times with ethyl acetate and filtered through celite. The solution was evaporated to very low volume and the residue was triturated with ethanol. This process was repeated until the product crystallised to give 9-O-(N-glycyl)-2-aminoethylclavulanic acid (479 mg) as pale yellow crystals with one mole of ethanol of crystallisation.

I.R. $\nu_{max}$ (KBr) 1783 and 1650 (very broad) $cm^{-1}$.
N.M.R. $\delta$ ($D_2O$), 3.10 (1H, d, J = 17Hz), 3.3—3.9 (7H, m), 4.14 (2H, d, J = 7Hz), 4.88 (1H, t, J = 7Hz), 4.96 (1H, s), 5.73 (1H, d, J = 3Hz).

Example 7

Benzyl 9-O-[2-(3-benzyloxycarbonyl)propionamido]ethylclavulanate

A solution of benzyl clavulanate (1.445 gm) and N-(3-benzyloxycarbonyl)propionyl aziridine (1.17 gm) in dry methylene chloride (25 ml) was cooled in a dry ice/acetone bath and treated with boron trifluoride etherate (0.1 ml). After ¾ hour the solution was allowed to warm to room temperature for 1½

hours. Excess sodium bicarbonate solution was then added and the organic phase separated. The aqeuous phase was extracted twice with chloroform and the combined organic phases were washed with brine, dried over magnesium sulphate, and evaporated. The product was isolated by column chromatography of the residue using gradient elution (Kieselgel 1:1 ethyl acetate:cyclohexane going to neat ethyl acetate) to yield benzyl 9-O-[2-(3-benzyloxycarbonyl)propionamido]ethylclavulanate (1.181 gm) m.p. 85—88° from cyclohexane/ethyl acetate.

I.R. $\nu_{max}$ (nujol) 3250, 1800, 1745, 1685 and 1650 $cm^{-1}$.
N.M.R. $\delta$ ($CDCl_3$) 2.35—2.8 (4H, m), 3.01 (1H, d, J = 17Hz), 3.3—3.6 (5H, m), 4.04 (2H, d, J = 7Hz), 4.78 (1H, broad t, J = 7Hz), 5.10 (3H, z), 5.18 (2H, s), 5.65 (1H, d, J = 3Hz), 5.98 (1H, broad), 7.33 (10H, s).

Analysis

| | | | |
|---|---|---|---|
| Found: | C, 64.56; | H, 5.79; | N, 5.46. |
| $C_{28}H_{30}N_2O_8$ requires: | C, 64.36; | H, 5.79; | N, 5.36. |

Example 8

Disodium 9-O-[2-(3-carboxy)propionamido]ethylclavulanate

A solution of benzyl 9-O-[2-(3-benzyloxycarbonyl)propionamido]ethylclavulanate (1.0 gm) in tetrahydrofuran (30 ml) was hydrogenated over 10% palladium charcoal (300 mgs) for 20 minutes. The solution was filtered through celite and the filter cake washed with tetrahydrofuran. The combined filtrates were diluted with water and treated with a solution of sodium bicarbonate (160 mgs) in water. Most of the tetrahydrofuran was removed on a rotary evaporator and the residual aqueous solution was extracted three times with ethyl acetate. The solution was then filtered through celite and freeze dried to yield disodium 9-O-[2-(3-carboxy)propionamido]ethylclavulanate (574 mgs).

I.R. $\nu_{max}$ (KBr) 1783, 1625 (very broad).
N.M.R. $\delta$ ($D_2O$) 2.3—2.7 (4H, m), 3.03 (1H, d, J = 17Hz), 3.15—3.65 (5H, m), 4.06 (2H, d, J = 8Hz), 4.83 (1H, broad t, J = 8Hz), 4.91 (1H, s), 5.67 (1H, d, J = 3Hz).

Example 9

9-O-(N-L-prolyl)-2-aminoethyl clavulanic acid

A solution of benzyl 9-O-[N'-benzyloxycarbonyl-L-prolyl)-2-aminoethyl clavulanate (0.500 gm) in tetrahydrofuran (10 ml) and water (5 ml) was hydrogenated over 10% palladium charcoal (0.170 gm) for 40 mins. The solution was filtered through celite and the filter cake was then washed with water. The tetrahydrofuran was removed on a rotary evaporator and the remaining aqueous solution was extracted three times with ethyl acetate. The solution was evaporated and the residue dried over phosphorus pentoxide. 9-O-(N-L-prolyl)-2-amino ethyl clavulanic acid was obtained in a yield of 0.243 gm.

I.R. $\nu_{max}$ (KBr) 1784, 1670 and 1628 $cm^{-1}$.
N.M.R. $\delta$ ($D_2O$) 1.8—2.5 (4H, m), 3.15 (1H, d, J = $17H_3$), 3.3—3.8 (7H, m), 4.10 (2H, d, J = 7Hz), 4.2—4.45 (1H, m), 4.84 (1H, broad t, J 7Hz), 4.92 (1H, s), 5.79 (1H, d, J = $3H_3$).

Example 10

Benzyl 9-O-[N-(N-benzyloxycarbonyl-D-alanyl)-2-aminoethyl clavulanate

A stirred solution of benzyl clavulanate (1.445 gm) and 1-(N-benzyloxycarbonyl)-D-alanyl aziridine (1.24 gm) in dry methylene chloride (25 ml) was cooled in a dry ice acetate bath and treated with boron trifluoride etherate (0.1 ml). After 1 hr the solution was allowed to warm to room temperature over a period of $2\frac{1}{2}$ hrs. Excess sodium bicarbonate solution was then added and the organic phase separated. The aqueous phase was extracted twice with chloroform, and the combined organic phases were washed with brine, dried over magnesium sulphate and evaporated. Benzyl 9-O-[N-(N'-benzyloxycarbonyl-D-alanyl)-2-aminoethyl]clavulanate (1.977 gm) was isolated by column chromatography using gradient elution (Kieselgel, 1:1 going to 2:1 ethyl acetate:cyclohexane).
$\alpha_D$ + 26.6°(c, 1 $CHC1_3$)

I.R. $\nu_{max}$ (nujol) 3260, 1802, 1741, 1695 and 1653 $cm^{-1}$.
N.M.R. $\delta$ ($CDC1_3$), 1.33 (3H, d, J = $7H_z$) 2.99 (1H, d, J = $17H_z$), 3.3—3.55 (5H, m), 4.02 (2H, d, J = $7H_z$), 4.0—4.35 (1H, m), 4.75 (1H, broad t, J = $7H_z$), 5.07 (3H, s), 5.17 (2H, s), 5.47 (1H, broad d, J = $7H_z$), 5.64 (1H, d, J = 3H), 6.35 (1H, broad s) 7.31 (10H, s).

Example 11

9-O-(N-D-alanyl)-2-aminoethyl clavulanic acid

A solution of benzyl 9-O-[N'-(N'-benzyloxycarbonyl-D-alanyl)-2-aminoethyl clavulanate (0.700 gm) in tetrahydrofuran (14 ml) and water (7 ml) was hydrogenated over 10% palladium charcoal

(0.250 gm) for 30 mins. The solution was filtered through celite and the filter cake then washed with water. Most of the tetrahydrofuran was removed on a rotary evaporator and the residual aqueous solution was washed three times with ethyl acetate and evaporated to low volume. Absolute ethanol was then added and the solution again evaporated to low volume. This process was repeated once more. Scratching the residual gum with absolute ethanol induced crystallisation. Solid 9-O-(N-D-alanyl)-2-aminoethyl clavulanic acid (0.179 gm) was filtered off and dried over phosphorus pentoxide.

I.R. $\nu_{max}$ (kBr) 1782, 1655 and 1600 (broad) $m^{-1}$.
N.M.R. $\delta$ ($D_2O$) 1.48 (3H, d, J = $7H_z$), 3.07 (1H, d, J = $17H_z$) 3.3—3.7 (5H, m), 3.99 (1H, q, J = $7H_z$), 4.09 (2H, d, J = $7H_z$), 4.85 (1H, broad t, J = $7H_z$), 4.91 (1H, s), 5.68 (1H, d, J = $3H_z$).

Example 12

Benzyl 9-O-[2-(3-benzyloxycarbonyl-2-benzyloxycarbonylamino)propionamido] ethyl clavulanate

A stirred solution of benzyl clavulanate (0.578 gm) and N-($\beta$-benzyl N'benzyloxycarbonyl)-$\alpha$-DL-aspartyl aziridine in dry methylene chloride (10 ml) was cooled in dry ice-acetone bath and treated with boron trifluoride etherate (0.04 ml). After $\frac{1}{2}$ hour the mixture was allowed to warm to room temperature over a period of 2· hrs. Excess sodium bicarbonate solution was then added and the organic phase separated. The aqueous phase was extracted three times with chloroform, and the combined organic phases were washed with brine, dried over magnesium sulphate and evaporated. Benzyl 9-O-[2-(3-benzyloxycarbonyl-2-benzyloxycarbonylamino) propioamido ethyl clavulanate (yield 0.811 gm) was isolated as a pale yellow gum by column chromatography of the residue using gradient elution (Kieselgel 2:1 going to 1:1 cyclohexane:ethyl acetate as eluent).

I.R. $\nu_{max}$ ($CHC1_3$), 3380, 1805, 1745, 1720 and 1670 $cm^{-1}$.
N.M.R. $\delta$ ($CDC1_3$), 2.5—3.0 (2H, m), 2.96 (1H, d, J = $17H_z$), 3.2—3.55 (5H, m), 3.99 (2H, d, J = $7H_z$), 4.45—4.85 (2H, m), 5.05 (2H, s), 5.14 (3h, s), 5.62 (1H, d, J = $3H_z$), 5.7—6.2 (2H, broad m) 7.28 (15 H, s).

Example 13

Sodium 9-O-(N-DL-$\alpha$-aspartyl)-2-aminoethyl clavulanate

A solution of benzyl 9-O-[2-(3-benzyloxycarbonyl-2-benzyloxycarbonylamino) propionamido]-ethyl clavulanate (0.753 gm) in tetrahydrofuran (10 ml) and water (5 ml) was hydrogenated over 10% palladium charcoal (0.250 gm) for 30 mins. The solution was filtered through celite and the filter cake washed with water. The combined filtrates were treated with a solution of sodium bicarbonate (94.2 mg) in water, and most of the tetrahydrofuran was removed on a rotary evaporator. The aqueous solution was extracted three times with ethyl acetate, filtered through celite, and evaporated. The residue, sodium 9-O-(N-DL-$\alpha$-aspartyl)-2-aminoethyl clavulanate (yield 0.351 gm) was dried over phosphorus pentoxide.

I.R. $\nu_{max}$ (KBr) 1780 and 1610 (very broad) $cm^{-1}$.
N.M.R. $\delta$ ($D_2O$) 2.55—3.1 (2H, m), 3.07 (1H, d, J = $17H_z$), 3.25—3.7 (5H, m), 3.85—4.2 (1H, m), 4.10 (2H, d, J = $7H_z$) 4.85 (1H, broad t, J = $7H_z$), 4.93 (1H, s), 5.70 (1H, d, J = $3H_z$).

Example 14

Benzyl 9-O-[N-(6-benzyloxycarbonylaminohexanoyl)-2-aminoethyl] clavulanate

A solution of benzyl clavulanate (1.734 gm) and N-(N'-benzyloxycarbonyl)-6-aminohexanoyl aziridine (1.740 gm) in dry methylene chloride (30 ml) was cooled in a dry-ice acetone bath, and treated with boron trifluoride etherate (0.12 ml). After 1 hr the mixture was allowed to warm to room temperature over a period of 2 hrs. Excess sodium bicarbonate solution was added and the organic phase separated. The aqueous phase was extacted three times with chloroform and the combined extracts were washed with water, then brine, and dried over magnesium sulphate. The solvent was evaporated and benzyl 9-O-[N-(6-benzyloxycarbonylaminohexanoyl)-2-aminoethyl] clavulanate (yield 1.981 gm) isolated by column chromatography of the residue using gradient elution (Kieselgel, 1:1 cyclohexane:ethyl acetate going to neat ethyl acetate).
m.pt 104—106° from ethyl acetate/cyclohexane.

I.R. $\nu_{max}$ (nujol) 3330, 1800, 1745, 1690 and 1640 $cm^{-1}$.
N.M.R. $\delta$($CDCl_3$) 1.1—1.8 (6H, m), 2.13 (2H, t, J = $7H_z$), 2.9—3.6 (8H, m), 4.04 (2H, d, J = $7H_z$), 4.78 (1H, t, J = $7H_z$), 4.95 (1H, broad), 5.07 (3H, s), 5.18 (2H, s), 5.67 (1H, d, J = $3H_z$), 5.85 (1H, broad) 7.33 (5H, s).
Analysis

Found: C, 64.29; H, 6.24; N, 7.47.
$C_{31}H_{37}N_3O_8$ requires: C, 64.22; H, 6.43; N, 7.25.

Example 15

9-O-[N-aminohexanoyl)-2-aminoethyl] clavulanate

A solution of benzyl 9-O-[N-(6-benzyloxycarbonylaminohexanoyl)-2-aminoethyl] clavulanate (1 gm) in tetrahydrofuran (20 ml) and distilled water (10 ml) was hydrogenated over 10% palladium charcoal (0.300 gm) for 3 mins. The solution was filtered through celite and the filter cake washed with water. Most of the tetrahydrofuran was removed on a rotary evaporator and the residual aqueous solution was extracted three times with ethyl acetate, filtered through celite and evaporated. The residue was dried over phosphorus pentoxide. 9-O-[N-(6-aminohexanoyl)-2-aminoethyl]clavulanate was obtained in a yield of 0.479 gm.

I.R. $\nu_{max}$ (KBr) 1784 and 1640 (broad) $cm^{-1}$.
N.M.R. $\delta$ ($D_2O$) 1.1—1.8 (6H, m) 2.22 (2H, t, J = 7$h_z$), 2.9—3.7 (8H, m), 4.06 (2H, d, J = 8$H_z$), 4.83 (1H, t, J = 8$H_z$) 4.91 (1H, s), 5.68 (1H, d, J = 3$H_z$).

Example 16

Benzyl 9-O-[N-(N'-benzyloxycarbonyl-$\beta$-alanyl)-2-aminoethyl]clavulanate

A solution of benzyl clavulanate (1.445 gm), and N-(N'-benzyloxycarbonyl)-$\beta$-alanyl aziridine (1.240 gm) in dry methylene chloride (25 ml) was cooled in a dry ice acetone bath, and treated with boron trifluoride etherate (0.1 ml). After 1 hr the mixture was allowed to warm to room temperature over a period of 1½ hrs. Excess sodium bicarbonate solution was then added and the organic phase separated. The aqueous phase was extracted twice with chloroform, and the combined organic phases were washed with water, then brine, and dried over magnesium sulphate. Benzyl 9-O-[N-(N'-benzyloxy-carbonyl-$\beta$-alanyl)-2-aminoethylclavulanate] (yield 1.707 gm) was isolated by column chromatography of the residue using gradient elution. (Kieselgel 1:1 cyclohexane: ethyl acetate going to neat ethyl acetate).
m.pt 94—96° from ethyl acetate/cyclohexane.

I.R. $\nu_{max}$ (nujol) 3270, 1795, 1740, 1695, 1645 $cm^{-1}$.
N.M.R. $\delta$ ($CDCl_3$) 2.36 (2H, t, J = 6$H_z$), 2.99 (1H, d, J = 17$H_z$), 3.3—3.6 (7H, m), 4.02 (2H, d, J = 7$H_z$), 4.76 (1H, t, J = 7$H_z$), 5.06 (3H, s), 5.17 (2H, s), 5.45 (1H, broad), 5.66 (1H, d, J = 3$H_z$) 5.88 (1H, broad), 7.32 (5H, s).

Analysis

| | | | |
|---|---|---|---|
| Found: | C, 62.94; | H, 5.94; | N, 7.53. |
| $C_{28}H_{31}N_3O_3$ requires: | C, 62.56; | H, 5.81; | N, 7.82. |

Example 17

9-O-(N-$\beta$-alanyl)-2-aminoethyl clavulanic acid

A solution of benzyl 9-O-[N-(N'-benzyloxycarbonyl-$\beta$-alanyl)-2-aminoethyl] clavulanate (1 gm) in tetrahydrofuran (20 ml) and water (10 ml) was hydrogenated over 10% palladium charcoal (0.300 gm) for 30 mins. The solution was filtered through celite and the filter cake washed with water. Most of the tetrahydrofuran was removed on a rotary evaporator and the residual aqueous solution was extracted three times with ethyl acetate, filtered through celite and evaporated. The residue was dried over phosphorus pentoxide. 9-O(N-$\beta$-alanyl)-2-aminoethyl clavulanic acid was obtained in a yield of 0.479 gm.

I.R. $\nu_{max}$ (KBr) 1782 and 1640 (broad) $cm^{-1}$.
N.M.R. $\delta$ ($D_2O$) 2.61 (2H, t, J = 7$H_z$), 3.04 (1H, d, J = 17$H_z$). 3.2—3.6 (7H, m), 4.07 (2H, d, J = 7$H_z$), 4.81 (1H, broad t, J = 7$H_z$), 4.81 (1H, s), 5.66 (1H, d, J = 3$H_z$).

Example 18

Benzyl 9-O-[N-(N'-benzyloxycarbonyl) phenylalanyl]-2-aminoethyl clavulanate

A solution of benzyl clavulanate (1.445 gm) and N-(N'-benzyloxycarbonyl)-phenylalanyl aziridine (1.620 gm) in dry methylene chloride (25 ml) was cooled in a dry ice/acetone bath and treated with boron trifluoride etherate (0.1 ml). After 1 hr the solution was allowed to warm to room temperature over a period of 1½ hrs. Excess sodium bicarbonate solution was then added and the organic phase separated. The aqueous phase was extracted twice with chloroform, and combined organic phases were washed with water, dried over magnesium sulphate, and evaporated. The residue was recrystallised from ethyl acetate-cyclohexane and benzyl 9-O-[N-(N'-benzyloxycarbonyl)phenylalanyl]-2-aminoethyl clavulanate was obtained in a yield of 2.094gm.
m.pt 120—133.

Analysis

| | | | |
|---|---|---|---|
| Found: | C, 66.46; | H, 5.61; | N, 6.71. |
| $C_{34}H_{35}N_3O_8$ requires: | C, 66.55; | H, 5.75; | N, 6.85. |

I.R. $\nu_{max}$ (nujol) 3330, 1800, 1750, 1693 and 1656 $cm^{-1}$.
N.M.R. $\delta$ ($CDCl_3$) 2.85—3.10 (3H, m), 3.2—3.55 (5H, m), 3.94 (2H, d, J = 7$H_z$), 4.36 (1H, q, J = 5$H_z$), 4.70 (1H, t, J = 7$H_z$) 5.05 (3H, s) 5.17 (2H, s), 5.48 (1H, broad d, J = 7$H_z$), 5.64 (1H, d, j = 3$H_z$), 6.05 (1H, broad s), 7.1—7.4 (15H, m).

## Example 19

9-O-(N-I-phenylalanyl)-2-aminoethylclavulanate

A solution of benzyl 9-O-[N-(N'-benzyloxycarbonyl) phenylalanyl]-2-aminoethylclavulanate (1 gm) in tetrahydrofuran (20 ml) and water 10 ml) was hydrogenated over 10% palladium charcoal (0.300 gm) for 20 mins. The solution was filtered through celite and the filter cake washed with water. Most of the tetrahydrofuran was then removed on a rotary evaporator. The aqueous solution was extracted three times with ethyl acetate, filtered through celite and evaporated to ~1 ml. Acetone was added and the solid filtered off, washed with acetone and dried over phosphorus pentoxide. 9-O-(N-L-phenylalanyl)-2-aminoethyl clavulanate was obtained in a yield of 0.494 gm.

Analysis

| | | | |
|---|---|---|---|
| Found: | C, 58.37; | H, 5.98; | N, 10.71. |
| $C_{19}H_{23}N_3O_6$ requires: | C, 58.60; | H, 5.95; | N, 10.79. |

I.R. $\nu_{max}$ (KBr), 1782, 1670, 1610 $cm^{-1}$.

## Example 20

Benzyl 9-O-[N-L-$\beta$-(N'-benzyloxycarbonyl, $\alpha$-benzyl)aspartyl]-2-aminoethyl clavulanate

A stirred solution of benzyl clavulanate (1.445 gm) and N-($\alpha$, benzyl-N'-benzyloxycarbonyl)-$\beta$-aspartyl aziridine (1.96 gm) in dry methylene chloride (25 ml) was cooled in a dry ice-acetone bath; and treated with boron trifluoride etherate (0.10 ml). After 1 hr the mixture was allowed to warm to room temperature over a period of 1 hr. Excess sodium bicarbonate solution was then added and the organic phase separated. The aqueous phase was extracted three times with chloroform and the combined organic phases were washed with water, then brine, dried over magnesium sulphate and evaporated. Benzyl 9-O-[N-L-$\beta$-(N'-benzyloxycarbonyl, $\alpha$-benzyl)aspartyl]2-aminoethyl clavulanate (yield 2,332 gm) was isolated by column chromatography of the residue using gradient elution (Kieselgel 3:1 cyclohexane-ethyl acetate going to neat ethyl acetate).
m.pt 115—119 from ethyl acetate/cyclohexane.
I.R. $\nu_{max}$ (nujol) 3330, 1805, 1740, 1695 and 1645 $cm^{-1}$.
N.M.R. $\delta$ ($CDCl_3$) 2.65—2.85 (2H, m), 2.98 (1H, d, J = 17$H_z$) 3.2—3.55 (5H, m), 4.00 (2H, d, J = 7$H_z$), 4.45—4.85 (2H, m), 5.08 (2H, s), 5.17 (3H, s), 5.64 (1H, d, J = 3$H_z$), 5.8—6.2 (2H, broad) 7.31 (15H, s).

## Example 21

Sodium 9-O-(N-L-aspartyl)-2-aminoethyl clavulanate

A solution of benzyl 9-O-N-L-(N'-benzyloxycarbonyl benzyl)aspartyl-2-aminoethyl clavulanate (1 gm) in tetrahydrofuran (20 ml) and water (10 ml) was hydrogenated over 10% palladium charcoal (0.300 gm) for 25 mins. The solution was filtered through celite and the filter cake washed with water. The combined filtrates were treated with a solution of sodium bicarbonate (0.123 gm) in distilled water. Most of the tetrahydrofuran was removed on a rotary evaporator and the residual aqueous solution was extracted three times with ethyl acetate. The aqueous solution was filtered through celite and evaporated. The residue was dried over phosphorus pentoxide. Sodium 9-O-(N-L-aspartyl)-2-aminoethylclavulanate was obtained in a yield of 0.518 gm.

I.R. $\nu_{max}$ (KBr), 1783 and 1620 (v. broad) $cm^{-1}$.
N.M.R. ($D_2O$) 2.7—2.90 (2H, m), 3.04 (1H, d, J = 17$H_z$), 3.2—3.65 (5H, m), 3.94 (1H, broad t, J = 7$H_z$), 4.09 (2H, d, J = 7$H_z$), 4.83 )1H, t, J = 7$H_z$) 4.91 (1H, s), 5.69 (1H, d, J = 3$H_z$).

*Description 1*

N-(2-benzyloxycarbonyl)acetyl aziridine

A solution of monobenzyl malonate (1.74 gm) in dry methylene chloride (20 ml) was cooled in an ice bath and treated successively with pyridine (0.74 ml) and aziridine (0.47 ml). A solution of dicyclohexylcarbodiimide (2.1 gm) in methylene chloride (20 ml) was then added over 30 minutes. The mixture was allowed to warm to room temperature over 2.5 hours and then filtered through celite. The filtrate was washed twice with citric acid solution (25 ml of 1N) then with sodium bicarbonate solution (25 ml of 1N) and finally with brine (20 ml). The solution was dried over magnesium sulphate and evaporated. The residue was dissolved in ethyl acetate, and the solution filtered and evaporated. The product (1.215 gm) was obtained by column chromatography of the residue (Kieselgel 2:1 cyclohexane:ethyl acetate).

I.R. $\nu_{max}$ (film 1750 and 1705 $cm^{-1}$.
N.M.R. $\delta$ ($CDCl_3$) 2.25 (4H, s), 3.53 (2H, s), 5.18 (2H, s), 7.37 (5H, s).

*Description 2*

1-(N-benzyloxycarbonyl-L-alanyl aziridine

A solution of N-benzyloxycarbonyl-L-alanine (2.23 gm) in dry methylene chloride (20 ml) was treated with pyridine (0.8 ml) and then cooled in an ice-bath. Aziridine (0.6 ml) was added. A solution of dicyclohexylcarbodiimide (2.2 gm) in dry methylene chloride (20 ml) was then added over 30 minutes. After warming to room temperature over 2 hours the solution was filtered through celite. The solution was washed with citric acid solution (3 x 25 ml, 1N) then with sodium bicarbonate solution (25 ml, 1N) and finally with brine (20 ml). The solution was dried over magnesium sulphate and evaporated. The residue dissolved in ethyl acetate and the solution was again filtered and evaporated. The product was purified by column chromatography (Kieselgel 1:1 ethyl acetate cyclohexane as eluent) to yield 1-(N-benzyloxycarbonyl)-L-alanyl aziridine, m.p. 82—83° (from ethyl acetate-cyclohexane).

I.R. $\nu_{max}$ (nujol) 3310 and 1703 (broad) $cm^{-1}$.
N.M.R. $\delta$ ($CDCl_3$) 1.56 (3H, d, J = 7Hz), 2.27 (4H, s), 4.40 (1H, broad quintet, J = 7Hz), 5.08 (2H, s), 5.45 (1H, broad), 7.33 (5H, s).

Analysis

| | | | |
|---|---|---|---|
| $C_{13}H_{16}N_2O_3$ requires: | C, 62.89; | H, 6.50; | N, 11.28. |
| Found: | C, 62.70; | H, 6.39; | N, 11.20. |

*Description 3*

1-(N-benzyloxycarbonyl)glycyl aziridine

A stirred solution of N-benzyloxycarbonyl-glycine (2.09 gm) in dry methylene chloride (20 ml) was treated with pyridine (0.8 ml) and cooled in an ice-bath. Aziridine (0.60 ml) was then added followed by a solution of dicyclohexylcarboxiimide (2.1 gm) in methylene chloride (20 ml) which was added over 20 minutes. The mixture was then allowed to warm to room temperature over 2.5 hours. The mixture was then filtered and the filtrate washed twice with citric acid solution, then with sodium bicarbonate solution, and finally with brine. the solution was dried over magnesium sulphate and evaporated. The product was isolated by column chromatography of the residue (Kieselgel 1:1 ethyl acetate:cyclohexane going to neat ethyl acetate) to yield 1-(N-benzyloxycarbonyl) glycyl aziridine (1.48 gm).

I.R. $\nu_{max}$ (film) 3330 and 1710 (broad) $cm^{-1}$.
N.M.R. $\delta$ ($CDCl_3$ 2.25 (4H, s), 4.06 (2H, d, J = 5Hz), 5.12 (2H, s), 5.53 (1H, v. broad), 7.35 (5H, s).

*Description*

N-(3-benzyloxycarbonyl)propionyl aziridine

A stirred solution of monobenzyl succinate (2.08 gm) in dry methylene chloride (20 ml) was treated with pyridine (0.8 ml), cooled in an ice bath, and then treated with aziridine (0.6 ml). A solution of dicyclohexylcarbodiimide (2.2 gm) in dry methylene chloride was then added over 20 minutes. The mixture was allowed to warm to room temperature for 3 hours. The solution was filtered through celite, washed twice with citric acid solution, once with sodium bicarbonate solution, and finally with brine. The solution was dried over magnesium sulphate and evaporated, the residue was dissolved in ethyl acetate, filtered and again evaporated. The product was isolated by column chromatography of the residue (Kieselgel 1:1 ethyl acetate:cyclohexane to yield N-(3-benzyloxycarbonyl)propionyl aziridine (1.18 gm).

I.R. $\nu_{max}$ (film) 1735 and 1695 $cm^{-1}$.
N.M.R. $\delta$ ($CDCl_3$) 2.22 (4H, s), 2.71 (4H, s), 5.11 (2H, s), 7.35 (5H, s).

*Description 5*

N-(N'-benzyloxycarbonyl)prolyl aziridine

A stirred solution of N-benzyloxycarbonyl proline (2.49 gm) in dry methylene chloride (20 ml) was cooled in an ice bath and treated with pyridine (0.8 ml) and aziridine (0.6 ml). A solution of dicyclohexylcarbodiimide (2.2 gm) in dry methylene chloride (20 ml) was then added over 20 mins, and the mixture was then allowed to warm to room temperature for $2\frac{1}{2}$ hrs. The solution was then filtered and washed twice with citric acid solution, once with sodium bicarbonate solution and finally with brine. The solution was dried over magnesium sulphate and evaporated. The residue was dissolved in ethyl acetate and the solution filtered and evaporated. N-(N'-benzyloxycarbonyl)prolyl aziridine (2.068 gm) was isolated by column chromatography of the residue (Kieselgel 2:1 cyclohexane = ethyl acetate as eluent) as a pale yellow gum.

I.R. $\nu_{max}$ (film) 3500 1700 (broad) cm.
N.M.R. ($CDCl_3$) 1.8—2.3 (4H, m), 2.35 (4H, m), 3.4—3.8 (2H, m), 4.3—4.6 (2H, m)5³.12(2H, s) 7.35 (5H, s).

*Description 6*

1-(N-benzyloxycarbonyl)-D-alanyl aziridine

A stirred solution of D-(N-benzyloxycarbonyl) alanine (2.23 gm) and pyridine (0.8 ml) in dry methylene chloride (20 ml) was cooled in an ice bath and treated with aziridine (0.6 ml). A solution of dicyclohexylcarbodiimide (2.2 gm) in dry methylene chloride (20 ml) was then added over 20 mins, and the mixture was then allowed to warm to room temperature over a period of 3 hrs. The solution was then filtered and washed twice with citric acid solution, once with sodium bicarbonate solution, and once with brine. The solution was dried over magnesium sulphate and evaporated. The residue was dissolved in ethyl acetate, the solution filtered and evaporated. 1-(N-benzyloxycarbonyl)-D-alanyl aziridine (1.42 gm) was isolated by column chromatography of the residue (Kieselgel 2:1 cyclohexane:ethyl acetate as eluent). $\alpha_D$ −14—5° (c, 1 $CHCl_3$),

I.R. $\nu_{max}$ (nujol) 3310 and 1703 $cm^{-1}$.

*Description 7*

N-($\beta$-benzyl N′-benzyloxycarbonyl)$\alpha$-DL- aspartyl aziridine

A stirred solution of $\beta$-benzyl N-benzyloxycarbonyl DL aspartic acid (3.57 gm), in dry methylene chloride (20 ml) was cooled in an ice bath and treated with pyridine (0.8 ml) and then aziridine (0.6 ml.). A solution of dicyclohexylcarbodiimide (2.2 gm) in dry methylene chloride (20 ml) was then added over a period of 20 mins. The mixture was then stirred overnight at room temperature. The solution was filtered and then washed twice with citric acid solution, once with sodium bicarbonate, and finally with brine. The solution was dried over magnesium sulphate and evaporated. The residue was dissolved in ethyl acetate and the solution filtered and evaporated. N-($\beta$-benzyl-N′-benzyloxycarbonyl)-$\alpha$-DL aspartyl aziridine (3.169 gm) was obtained by chromatography of the residue (Kieselgel 1:1 cyclohexane, ethyl acetate).

m.pt 78—79° from cyclohexane/ethyl acetate.

I.R. $\nu_{max}$ (nujol) 3320, 1750, and 1695 $cm^{-1}$.

N.M.R. ($CDCl_3$) 2.13 (4H, s), 2.9—3.15 (2H, m), 4.35—4.85 (1H, m), 5.10 (2H, s), 5.15 (2H, s), 7.33 (10H, s).

*Description 8*

N-N′-benzyloxycarbonyl)-6-aminohexanoyl aziridine

A solution of N-benzyloxycarbonyl-6-aminohexanoic acid (2.65 gms) and pyridine (0.8 ml) in dry methylene chloride (20 ml) was cooled in an ice bath and treated with aziridine (0.6 ml). A solution of dicyclohexylcarbodiimide (2.2 gm) in dry methylene chloride (20 ml) was then added over 20 mins. The mixture was then allowed to warm to room temperature over a period of 2 hrs. The solution was then filtered, washed twice with citric acid solution, once with sodium bicarbonate solution and finally with brine. The solution was dried over magnesium sulphate and evaporated. N-(N′-benzyloxycarbonyl)-6-aminohexanoyl aziridine (yield 1.831 gm) was isolated by column chromatography of the residue (Kieselgel 2:1 cyclohexane:ethyl acetate).

I.R. $\nu_{max}$ (film) 3300, 1700 (broad) $cm^{-1}$.

N.M.R. $\delta$ ($CDCl_3$) 1.2—1.9 (6H, m), 2.17 (4H, s) 2.39 (2H, t, J = 7Hz), 3.17 (2H, q, J = 7Hz), 4.98 (1H, broad), 5.10 (2H, s), 7.33 (5H, s).

*Description 9*

N-(N′-benzyloxycarbonyl)-$\beta$-alanyl aziridine

A stirred solution of N-benzyloxycarbonyl-$\beta$-alanine (2.23 gm) and pyridine (0.8 ml) in dry methylene chloride (20 ml) was cooled in an ice bath and treated with aziridine (0.6 ml). A solution of dicyclohexylcarbodiimide (2.2 gm) in dry methylene chloride (20 ml) was then added over 20 mins. When the addition was complete the mixture was allowed to warm to room temperature over a period of 2 hrs. The solution was filtered and washed twice with citric acid solution, once with sodium bicarbonate solution and once with brine. The solution was dried over magnesium sulphate and evaporated. N-(N′-benzyloxycarbonyl)-$\beta$-alanyl aziridine (yield 1.364 gm) was isolated by column chromatography of the residue (Kieselgel 2:1 cyclohexane:ethyl acetate)

I.R. $\nu_{max}$ (film). 3300, 1700 $cm^{-1}$.

N.M.R. $\delta$ ($CDCl_3$) 2.18 (4H, s), 2.63 (2H, t, J = 6$H_z$), 3.47 (2H, q, J = 6Hz) 5.10 (2H, s), 5.4 (1H, broad), 7.34 (5H, s).

*Description 10*

N-(N′-benzyloxycarbonyl)phenylalanyl aziridine

A stirred mixture of N-benzyloxycarbonyl-L-phenylalanine (2.99 gm) and pyridine (0.8 ml) in dry methylene chloride (20 ml) was cooled in an ice bath and treated with aziridine (0.6 ml). A solution of dicyclohexylcarbodiimide (2.2 gm) in dry methylene chloride (20 ml) was then added over 20 mins.

When the addition was complete the mixture was allowed to warm to room temperature over a period of 2 hrs. The solution was then filtered, washed twice with citric acid solution, once with sodium bicarbonate solution and finally with brine. The solution was dried over magnesium sulphate and evaporated. The residue was dissolved in ethyl acetate and the solution filtered and evaporated. N-(N'-benzyloxycarbonyl)phenylalanyl aziridine was isolated by column chromatography of the residue (Kieselgel 1:1 cyclohexane:ethyl acetate). The product was recrystallised from cyclohexane and a yield of 2.52 gm was obtained.
m.pt. 86—87°.

Analysis

| | | | |
|---|---|---|---|
| Found: | C, 70.12; | H, 6.13; | N, 8.50. |
| $C_{19}H_{20}NO_3$ requires: | C, 70.35; | H, 6.21; | N, 8.64. |

I.R. $\nu_{max}$ (nujol) 3380, 1696 $cm^{-1}$.
N.M.R. ($CDCl_3$) 2.13 (4H, s), 3.03 (2H, d, J = 6Hz), 4.45—4.95 (1H, m) 5.05 (2H, s), 5.35 (2H, broad d, J = 7Hz), 7.2—7.4 (10H).

*Description 11*

N-($\alpha$-benzyl, N'-benzyloxycarbonyl)-$\beta$-aspartyl aziridine

A stirred solution of $\alpha$-benzyl, N-benzyloxycarbonyl aspartic acid (3.57 gm), in dry methylene chloride (20 ml) was treated with pyridine (0.8 ml), cooled in an ice bath and treated with aziridine (0.6 ml). A solution of dicyclohexylcarbodiimide (2.2 gm) in dry methylene chloride (20 ml) was then added over 20 mins. The mixture was then stirred at room temperature for 2 hrs. The solution was filtered through celite, and washed twice with 1N citric acid solution, then once with water, once with sodium bicarbonate solution, and finally with brine. The solution was dried over magnesium sulphate and evaporated. The residue was dissolved in ethyl acetate and filtered. The solution was evaporated and N-($\alpha$-benzyl, N'-benzyloxycarbonyl)-$\beta$-aspartyl aziridine (yield 2.92 gm) isolated by column chromatography of the residue using gradient elution (Kieselgel, 3:1 going to 1:1 cyclohexane:ethyl acetate). The product was recrystallised from ethyl acetate/cyclohexane to give 2.09 gm.
m.pt 64—67°.

Analysis

| | | | |
|---|---|---|---|
| Found: | C, 66.15; | H, 6.02; | N, 7.39. |
| $C_{21}H_{22}N_2O_5$ requires: | C, 65.96; | H, 5.80; | N, 7.33. |

I.R. $\nu_{max}$ (nujol) 3300, 1740 and 1685 $cm^{-1}$.
N.M.R. $\delta$ ($CDCl_3$) 2.13 (4H, s) 2.98—3.15 (2H, m), 4.68 (1H, quintet, J = 4Hz), 5.13 (2H, s) 5.17 (2H, s) 5.96 (1H, broad d, J = 9Hz), 7.36 (5H, s).

*Demonstration of Activity*

a) Synergistic effect of 9-O-(N-glycyl)-2-aminoethylclavulanic acid

The MICs of ampicillin, 9-O-(N-glycyl)-2-aminoethylclavulanic acid (GAECA) alone and in admixture against a number of bacteria were determined by a standard microtitre method. The results obtained were as follows:

| Compound | MIC ($\mu$g/ml) | | | |
|---|---|---|---|---|
| | Staph. aureus Russell | Klebsiella aerog. E.70 | Proteus C 889 | E. coli JT 39 |
| Ampicillin alone | 250 | 500 | 2000 | 2000 |
| GAECA alone | 8 | 62.5 | 250 | 51.2 |
| Ampicillin + 5 $\mu$g/ml GAECA | 0.15 | 1.5 | 2 | 8 |
| Ampicillin + 1 $\mu$g/ml GAECA | 1.25 | 6.25 | 8 | 31.2 |

b) Synergistic effect of 9-O-(N-alanyl)-2-aminoethylclavulanic acid

The MICs of ampicillin, 9-O-(N-alanyl)-2-aminoethylclavulanic acid (AAECA) alone and in

admixture against a number of bacteria were determined by a standard microtitre method. The results obtained were as follows:

| Compound | MIC (μg/ml) | | | |
|---|---|---|---|---|
| | Staph. aureus Russell | Klebsiella aerog. E.70 | Proteus C 889 | E. coli JT 39 |
| Ampicillin alone | 500 | 500 | 1000 | 2000 |
| AAECA alone | 8 | 125 | 250 | 16 |
| Ampicillin + 5 μg/ml AAECA | — | 1.5 | 2 | 4 |
| Ampicillin + 1 μg/ml AAECA | 0.3 | 6.25 | 8 | 16 |

(c) Synergistic Activity of 9-O-(N-L-alanyl)-2-aminoethylclavulanic acid

The L-alanyl amino ethyl ether has good activity *in vivo* by both the oral and sub-cutaneous route. The results obtained were as follows:

*Synergistic activity in Mice infected intraperitoneally with E.Col: E96 (R* + strain)

| Compound | Route s/c | | Route p/o | |
|---|---|---|---|---|
| | Dosage mg/kg 5 | 2 | Dosage mg/kg 10 | 20 |
| Amoxycillin alone | >1000 | >1000 | >1000 | |
| Amoxycillin + Compound of Example 4 | 8.7 | 35 | 30 | — |
| Amoxycillin + Sodium Clavulanate | 13 | 52 | — | 60 |

Dosing at 1 and 5 hours post-infection s/c and 1, 3 and 5 hours post-infection p/o.

**Claims**

1. A compound of formula (I)

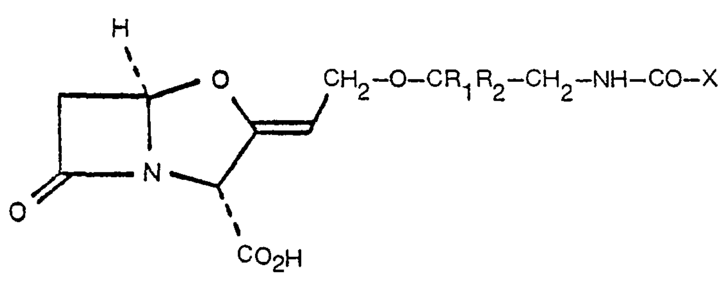

(I)

or a pharmaceutically acceptable salt or ester thereof wherein $R_1$ is a hydrogen atom or methyl group; $R_2$ is a hydrogen atom or methyl group; and X is a group of the sub-formula (a), (b), (c) or (d).

(a) $-CHR_3R_4$ (b) $-CH_2-CHR_3R_4$ (c) $-CH_2-CH_2-CHR_3R_4$ (d) 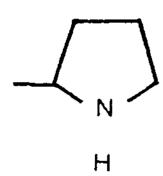

wherein $R_3$ is a hydrogen atom or a lower alkyl, aryl, aralkyl or substituted alkyl group; and $R_4$ is an

amino group or a carboxylic acid group, in which definitions the term "lower alkyl" means a group of up to 4 carbon atoms, the term "aryl" means a phenyl group or a phenyl group substituted by a fluorine or chlorine atom or a methoxyl, methyl or hydroxyl group, the term "aralkyl" means a lower alkyl group substituted by an aryl group, and the term "substituted alkyl" means a lower alkyl group substituted by an amino, carboxamide or carboxylic acid group.

2. A compound of the formula (I) according to claim 1 or a pharmaceutically acceptable salt thereof.

3. A compound according to claims 1 or 2 wherein $R_1$ is a hydrogen atom and $R_2$ is a hydrogen atom.

4. A compound according to any claims 1 to 3 wherein the compound of the formula (I) is of formula (II):

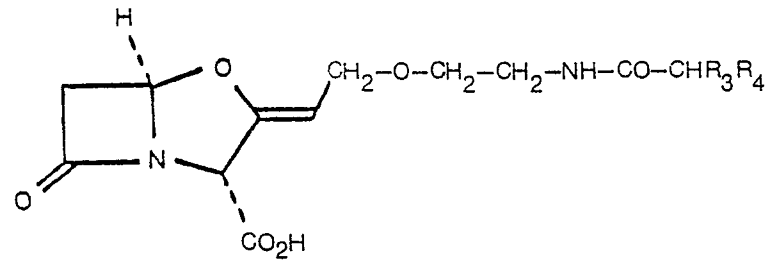

(II)

wherein $R_3$ and $R_4$ are as defined in claim 1.

5. A compound according to claims 2 or 3 wherein the compound of the formula (I) is of formula (III)

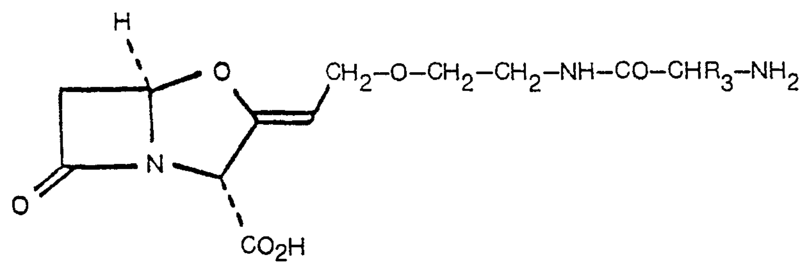

(III)

wherein $R_3$ is as defined in claim 1.

6. A compound according to any claims 1 to 3 wherein the compound of the formula (I) is of formula (IV):

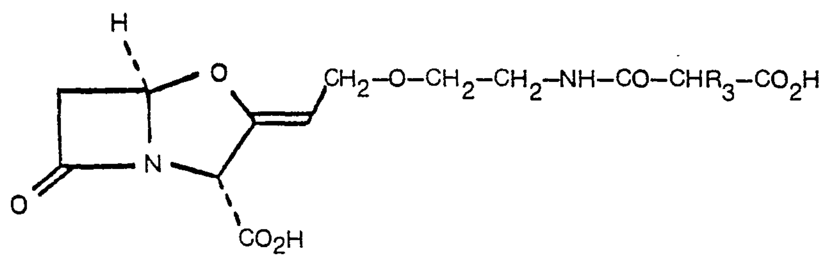

(IV)

wherein $R_3$ is as defined in claim 1.

7. A compound as claimed in any of claims 1 to 6 wherein $R_3$ is a hydrogen atom or a methyl, ethyl, n-propyl, isopropyl, phenyl, p-hydroxyphenyl, p-chlorophenyl, p-methoxyphenyl, benzyl, carboxymethyl, or 5-$(CH_2)_3$—$CH_2NH_2$ group.

8. An ester of a compound of the formula (I), (II), (III) or (IV) as claimed in any of claims 1 to 6, said ester having the part-formula (a) to (d):

(a) —CO—O—$A_1$ (b) —CO—O—$CH_2A_2$—O—$COA_3$

(c) 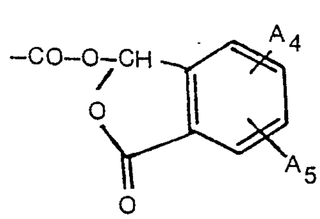 (d) —CO—O—$CHA_6A_7$

wherein $A_1$ is a lower alkyl, lower alkenyl, lower alkynyl, or a lower alkyl group substituted by a lower

alkoxyl, lower acyl, $CF_3$, $CCl_3$, $CO.C_6H_5$ or carboxylic acid group or a salt or lower alkyl ester thereof; $A_2$ is a hydrogen atom or a methyl group; $A_3$ is a lower alkyl, lower alkoxyl, phenyl or benzyl group; $A_4$ is a hydrogen atom or a methyl or methoxyl group; $A_5$ is a hydrogen atom or a methyl or a methoxyl group; $A_6$ is a phenyl group or a phenyl group substituted by a fluorine, chlorine, or bromine atom or a methyl, methoxyl or nitro group; and $A_7$ is a hydrogen atom or a phenyl group or a phenyl group substituted by a fluorine, chlorine or bromine atom or a methyl, methoxyl or nitro group.

9. A compound according to claim 1 wherein the $CHR_3R_4$ moiety in the compounds of the formulae (I)—(IV) is the R-isomer of the S-isomer.

10. Benzyl 9-O-2-(2-benzyloxycarbonyl) acetamido ethyl-clavulanate.

11. Disodium 9-O-2-(2-carboxy) acetamido ethyl-clavulanate.

12. Benzyl 9-O[N-(N'-benzyloxycarbonyl-L-alanyl)-2-aminoethyl]clavulanate.

13. 9-O-[N-L-alanyl]-2-aminoethyl clavulanic acid.

14. Benzyl 9-O-[N-(N'-benzyloxycarbonyl glycyl)-2-aminoethyl]clavulanate.

15. 9-O-(N-Glycyl)-2-aminoethyl clavulanic acid.

16. Benzyl 9-O-[2-(3-benzyloxycarbonyl) propionamide]ethyl clavulanate.

17. Disodium 9-O-[2-(3-carboxy) propionamido]ethyl clavulanate.

18. 9-O-(N-L-prolyl)-2-aminoethyl clavulanic acid.

19. Benzyl 9-O-[N-(N'-benzyloxycarbonyl-D-alanyl)-2-aminoethyl]clavulanate.

20. 9-O-(N-D-alanyl)-2-aminoethyl clavulanic acid.

21. Benzyl 9-O-[2-(3-benzyloxycarbonyl-2-benzyloxycarbonylamino) propionamido]ethyl clavulanate.

22. Sodium 9-O-(N-DL-$\alpha$-aspartyl)-2-aminoethyl clavulanate.

23. Benzyl 9-O-[N-(6-benzyloxycarbonyl aminohexanoyl)-2-aminoethyl]clavulanate.

24. 9-O-[N-(6-aminohexanoyl)-2-aminoethyl]clavulanate.

25. Benzyl 9-O-[N-(N'-benzyloxycarbonyl-$\beta$-alanyl)-2-aminoethyl]clavulanate.

26. 9-O-(N-$\beta$-alanyl)-2-aminoethyl clavulanic acid.

27. Benzyl 9-O-[N-(N'-benzyloxycarbonyl phenyl alanyl)-2-aminoethyl] clavulanate.

28. 9-O-(N-L-phenylalanyl)-2-aminoethyl clavulanate.

29. Benzyl 9-O-[N-L-$\beta$-(N'-benzyloxycarbonyl-$\alpha$-benzyl) aspartyl]-2-aminoethyl clavulanate.

30. Sodium 9-O-(N-L-$\beta$-aspartyl)-2-aminoethyl clavulanate.

31. Benzyl 9-O-N-(N'-benzyloxycarbonyl-L-prolyl)-2-aminoethyl clavulanate.

32. A process for the preparation of a compound of the formula (I) as claimed in claim 1 or a pharmaceutically acceptable salt or ester thereof which process comprises the deprotection of a compound of the formula (V):

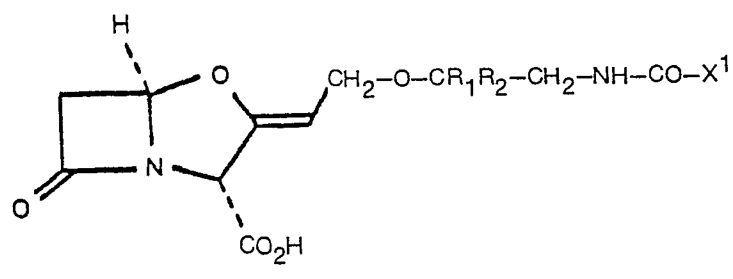

(V)

or a salt or ester thereof wherein $R_1$, $R_2$ and $R_3$ are as defined in claim 1 and $X^1$ is a group of the sub-formula (a), (b), (c) or (d) in which the amino or carboxylic group is protected.

33. A process for the preparation of a compound of the formula (V) as defined in claim 10 or a salt or ester thereof which process comprises the reaction of a compound of the formula (VI):

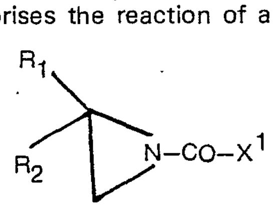

(VI)

wherein $R_1$, $R_2$, $R_3$, and $X^1$ are as defined in relation to formula (V), with an ester of clavulanic acid and thereafter if desired de-esterifying the resulting ester of the compound of the formula (V) to yield the compound of the formula (V) or its salt.

34. A pharmaceutical composition comprising a compound as claimed in any of claims 1 to 31 thereof together with a pharmaceutically acceptable carrier.

35. A pharmaceutical composition according to claim 34 which also comprises a penicillin or cephalosporin, the weight ratio of the compound of the formula (I) or its pharmaceutically acceptable salt or ester to penicillin or cephalosporin being 1:1 to 1:8.

36. A pharmaceutical composition according to claim 35 which comprises 25—500 mg of a

compound of formula (I) or its pharmaceutically acceptable salt and 150—1000 mg of amoxycillin, ampicillin or a prodrug thereof.

37. A pharmaceutical composition according to any of claims 34 to 36 comprising ampicillin trihydrate or amoxycillin trihydrate.

**Patentansprüche**

1. Eine Verbindung der Formel (I)

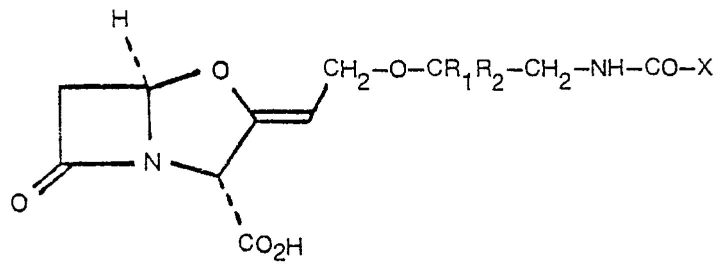 (I)

oder ein pharmakologisch verträgliches Salz oder ein Ester derselben, wobei $R_1$ ein Wasserstoffatom oder eine Methylgruppe ist, $R_2$ ein Wasserstoffatom oder eine Methylgruppe ist und X ein Rest der Unterformeln (a), (b), (c) oder (d) ist

(a) $—CHR_3R_4$ (b) $—CH_2—CHR_3R_4$ (c) $—CH_2—CH_2—CHR_3R_4$ (d) 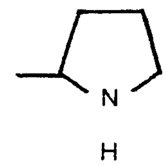

wobei $R_3$ ein Wasserstoffatom oder ein niederer Alkyl-, Aryl-, Arylalkyl- oder substituierter Alkylrest ist und $R_4$ eine Aminogruppe oder eine Carbonsäuregruppe ist, wobei in diesen Definitionen der Ausdruck "nieder-Alkyl" einen Rest mit bis zu 4 Kohlenstoffatomen meint, der Ausdruck "Aryl" einen Phenylrest oder einen mit einem Fluor- oder Chloratom oder einer Methoxy-, Methyl- oder Hydroxylgruppe substituierten Phenylrest meint, der Ausdruck "Arylalkyl" einen niederen Alkylrest, der mit einem Arylrest substituiert ist, meint und der Ausdruck "substituierter Alkyrest" einen niederen Alkylrest meint, der mit einer Amino-, Carboxamid- oder Carbonsäuregruppe substituiert ist.

2. Eine Verbindung der Formel (I) gemäß Anspruch 1 oder ein pharmakologisch verträgliches Salz derselben.

3. Eine Verbindung gemäß Anspruch 1 oder 2, wobei $R_1$ ein Wasserstoffatom und $R_2$ ein Wasserstoffatom ist.

4. Ein Verbindung gemäß einem der Ansprüche 1 bis 3, wobei die Verbindung der Formel (I) die Formel (II) hat:

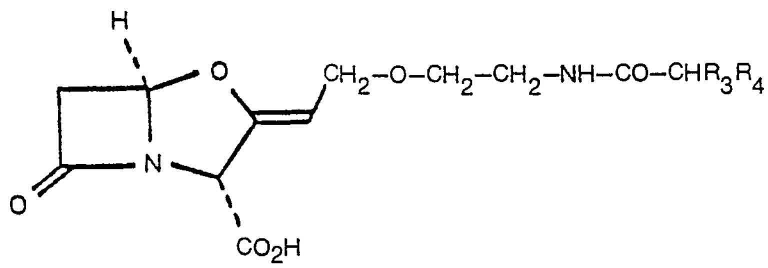 (II)

in der $R_3$ und $R_4$ wie in Anspruch 1 definiert sind.

5. Eine Verbindung gemäß Anspruch 2 oder 3, wobei die Verbindung der Formel (I) die Formel (III) hat:

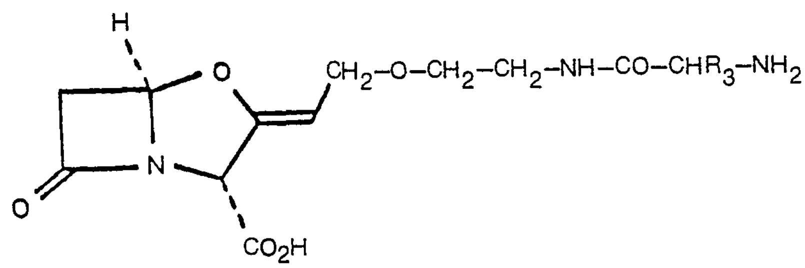 (III)

in der $R_3$ wie in Anspruch 1 definiert ist.

6. Eine Verbindung gemäß einem der Ansprüche 1 bis 3, wobei die Verbindung der Formel (I) die Formel (IV) hat:

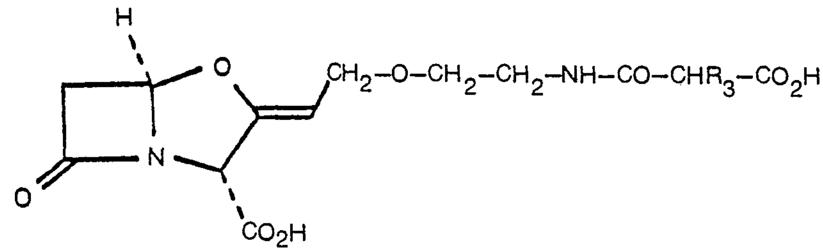 (IV)

in der $R_3$ wie in Anspruch 1 definiert ist.

7. Eine Verbindung gemäß einem der Ansprüche 1 bis 6, wobei $R_3$ ein Wasserstoffatom oder ein Methyl-, Äthyl, n-Propyl-, Isopropyl-, Phenyl-, p-Hydroxyphenyl-, p-Chlorphenyl-, p-Methoxyphenyl-, Benzyl-, Carboxymethyl-, oder 5-$(CH_2)_3$—$CH_2NH_2$-Rest ist.

8. Ein Ester einer Verbindung der Formel (I), (II), (III) oder (IV) gemäß einem der Ansprüche 1 bis 6, wobei der Ester die Teilformeln (a) bis (d) hat:

(a) $—CO—O—A_1$ (b) $—CO—O—CH_2A_2—O—COA_3$

(c) 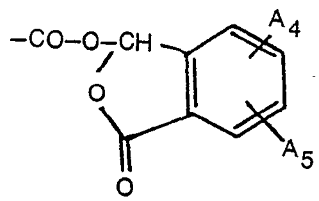 (d) $—CO—O—CHA_6A_7$

wobei $A_1$ ein niederer Alkylrest, niederer Alkenylrest, niederer Alkinylrest oder ein niederer Alkylrest ist, der mit einem niederen Alkoxyrest, niederen Acylrest, $CF_3$, $CCl_3$, $CO.C_6H_5$ oder einer Carbonsäuregruppe oder einem Salz oder einem niederen Alkylester derselben substituiert ist, $A_2$ ein Wasserstoff atom oder eine Methylgruppe ist, $A_3$ ein niederer Alkylrest, niederer Alkoxyrest, Phenyl- oder Benzylrest ist, $A_4$ ein Wasserstoffatom oder eine Methyl- oder Methoxygruppe ist, $A_5$ ein Wasserstoffatom oder eine Methyl- oder Methoxygruppe ist, $A_6$ ein Phenylrest oder ein mit einem Fluor-, Chlor- oder Bromatom oder einer Methyl-, Methoxy- oder Nitrogruppe substituierter Phenylrest ist, und $A_7$ ein Wasserstoffatom oder ein Phenylrest oder ein mit einem Fluor-, Chlor- oder Bromatom oder einer Methyl-, Methoxy- oder Nitrogruppe substituierter Phenylrest ist.

9. Eine Verbindung gemäß Anspruch 1, wobei der $CHR_3R_4$-Rest in den Verbindungen der Formeln (I) bis (IV) das R-Isomere oder das S-Isomere ist.

10. Benzyl-9-O-2-(2-benzoxycarbonyl)-acetamido-äthyl-clavulanat.

11. Dinatrium-9-O-2-(2-carboxy)-acetamido-äthyl-clavulanat.

12. Benzyl-9-O-[N-(N'-benzyloxycarbonyl-L-alanyl)-2-aminoäthyl]-clavulanat.

13. 9-O-[N-L-alanyl]-2-aminoäthyl-clavulansäure.

14. Benzyl-9-O-[N-(N'-benzyloxycarbonyl-glycyl)-2-aminoäthyl]-clavulanat.

15. 9-O-(N-Glycyl)-2-aminoäthyl-clavulansäure.

16. Benzyl-9-O-[2-(3-benzyloxycarbonyl)-propionamido]-äthyl-clavulanat.

17. Dinatrium-9-O-[2-(3-carboxy)-propionamido]-äthyl-clavulanat.

18. 9-O-(N-L-Prolyl)-2-aminoäthyl-clavulansäure.

19. Benzyl-9-O-[N-(N'-benzyloxycarbonyl-D-alanyl)-2-aminoäthyl]-clavulanat.

20. 9-O-(N-D-alanyl)-2-aminoäthyl-clavulansäure.

21. Benzyl-9-O-[2-(3-benzyloxycarbonyl-2-benzyloxycarbonylamino)-propionamido]-äthyl-clavulanat.

22. Natrium-9-O-(N-DL-$\alpha$-aspartyl)-2-aminoäthyl-clavulanat.

23. Benzyl-9-O-[N-(6-benzyloxycarbonyl-aminohexanoyl)-2-aminoäthyl]-clavulanat.

24. 9-O-[N-(6-aminohexanoyl)-2-aminoäthyl]-clavulanat.

25. 9-O-[N-(N'-benzyloxycarbonyl-$\beta$-alanyl)-2-aminoäthyl]-clavulanat.

26. 9-O-(N-$\beta$-alanyl)-2-aminoäthyl-clavulansäure.

27. Benzyl-9-O-[N'-(N-benzyloxycarbonyl-phenylalanyl]-2-aminoäthyl-clavulanat.

28. 9-O-(N-L-phenylalanyl)-2-aminoäthyl-clavulanat.

29. Benzyl-9-O-[N-L-$\beta$-(N'-benzyloxycarbonyl-$\alpha$-benzyl)-aspartyl]-2-aminoäthyl-clavulanat.

30. Natrium-9-O-(N-L-$\beta$-aspartyl)-2-aminoäthyl-clavulanat.

31. Benzyl-9-O-N-(N'-benzyloxycarbonyl-L-prolyl)-2-aminoäthyl-clavulanat.

32. Ein Verfahren zur Herstellung einer Verbindung der Formel (I) gemäß Anspruch 1 oder eines

pharmakologisch verträglichen Salzes oder Esters derselben, die Maßnahme umfassend, daß aus einer Verbindung der Formel (V):

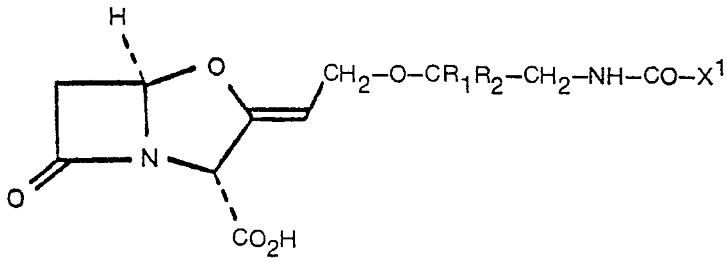

(V)

oder einem Salz oder Ester derselben, in der $R_1$, $R_2$ und $R_3$ wie in Anspruch 1 definiert sind und X' ein Rest der Unterformeln (a), (b), (c) oder (d) ist, in denen die Amino- oder Carbonsäuregruppe geschützt ist, die Schutzgruppe abgespalten wird.

33. Ein Verfahren zur Herstellung einer Verbindung der Formel (V) gemäß Anspruch 10 oder eines Salzes oder Esters derselben, die Maßnahmen umfassend, daß eine Verbindung der Formel (VI):

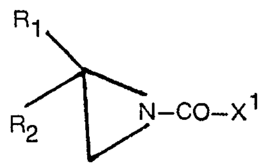

(VI)

in der $R_1$, $R_2$, $R_3$ und X' wie in bezug auf Formel (V) definiert sind, mit einem Ester der Clavulansäure umgesetzt wird und anschließend gegebenenfalls der entstandene Ester der Verbindung der Formel (V) zu einer Verbindung der Formel (V) oder deren Salz verseift wird.

34. Eine pharmazeutische Zusammensetzung, enthaltend eine Verbindung gemäß einem der Ansprüche 1 bis 31 zusammen mit einem pharmakologisch verträglichen Träger.

35. Eine pharmazeutische Zusammensetzung gemäß Anspruch 34, die auch ein Penicillin oder Cephalosporin enthält, wobei das Gewichtsverhältnis der Verbindung der Formel (I) oder deren pharmakologisch verträglichen Salzes oder Esters zu Penicillin oder Cephalosporin 1:1 bis 1:8 ist.

36. Eine pharmazeutische Zusammensetzung gemäß Anspruch 35, die 25 bis 500 mg einer Verbindung der formel (I) oder deren pharmakologisch verträglichen Salzes und 150 bis 1000 mg Amoxycillin, Ampicillin oder eine Vorstufe derselben enthält.

37. Eine pharmazeutische Zusammensetzung gemäß einem der Ansprüche 34 bis 36, Ampicillintrihydrat oder Amoxycillintrihydrate enthaltend.

**Revendications**

1. Composé de formule (I):

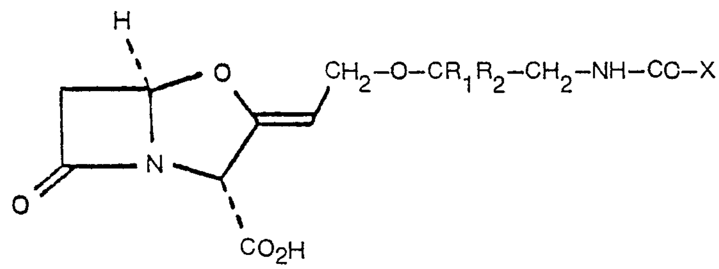

(I)

ou sel ou ester pharmaceutiquement acceptable de celui-ci formule dans laquelle $R_1$ est un atome d'hydrogéne ou un groupe méthyle; $R_2$ est un atome d'hydrogéne ou un groupe méthyle; et X est un groupe de sous-formule (a), (b), (c) ou (d):—

(a) $—CHR_3R_4$ (b) $—CH_2—CHR_3R_4$ (c) $—CH_2—CH_2—CHR_3R_4$ (d) 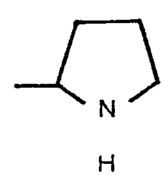

dans lesquelles $R_3$ est un atome d'hydrogène ou un groupe alcoyle inférieur, aryle, aralcoyle ou alcoyle substitué et $R_4$ est un groupe amino ou un groupe acide carboxylique, définitions dans lesquelles

l'expression "alcoyle inférieur" désigne un groupe ayant jusqu'à 4 atomes de carbone, le terme "aryle" désigne un groupe phényle ou bien un groupe phényle substitué par un atome de fluor ou de chlore ou par un groupe méthoxy, méthyle ou hydroxy, le terme "aralcoyle" désigne un groupe alcoyle inférieur substitué par un groupe aryle, et l'expression "alcoyle substitué" désigne un groupe alcoyle inférieur substitué par un groupe amino, carboxamide ou acide carboxylique.

2. Composé de formule (I) suivant la revendication 1 ou sel pharmaceutiquement acceptable de celui-ci.

3. Composé suivant la revendication 1 ou 2, caractérisé en ce que $R_1$ est un atome d'hydrogène et $R_2$ est un atome d'hydrogène.

4. Composé suivant l'une quelconque des revendications 1 à 3, caractérisé en ce que le composé de formule (I) répond à la formule (II):—

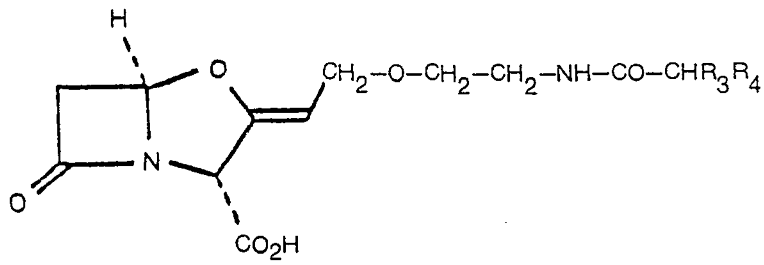 (II)

dans laquelle $R_3$ et $R_4$ ont la même signification que dans la revendication 1.

5. Composé suivant la revendication 2 ou 3, caractérisé en ce que le composé de formule (I) répond à la formule (III):—

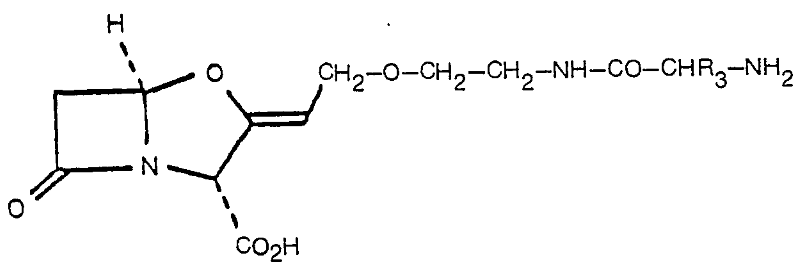 (III)

dans laquelle $R_3$ a la même signification que dans la revendication 1.

6. Composé suivant l'une quelconque des revendications 1 à 3, caractérisé en ce que le composé de formula (I) répond à la formule (IV):—

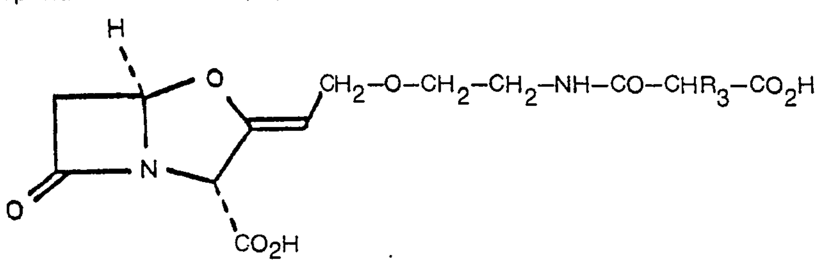 (IV)

dans laquelle $R_3$ a la même signification que dans la revendication 1.

7. Composé suivant l'une quelconque des revendications 1 à 6, dans lequel $R_3$ est un atome d'hydrogène ou un groupe méthyle, éthyle, n-propyle, isopropyle, phényle, p-hydroxyphényle, p-chlorophényle, p-méthoxyphényle, benzyle, carboxyméthyle ou 5-$(CH_2)_3$—$CH_2CH_2$.

8. Ester d'un composé de formule (I), (II), (III) ou (IV) suivant l'une quelconque des revendications 1 à 6, cet ester renfermant la formule partielle (a) à (d):

(a) $—CO—O—A_1$ (b) $—CO—O—CH_2A_2—O—COA_3$

(c) 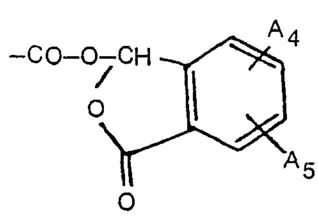 (d) $—CO—O—CHA_6A_7$

dans lesquelles $A_1$ est un groupe alcoyle inférieur, alcényle inférieur, alcynyle inférieur ou un groupe

alcoyle inférieur substitué par un groupe alcoxy inférieur, acyle inférieur, $CF_3$, $CCl_3$, $CO.C_6H_5$ ou acide carboxylique ou un sel ou ester d'alcoyle inférieur de celui-ci; $A_2$ est un atome d'hydrogène ou un groupe méthyle; $A_3$ est un groupe alcoyle inférieur, alcoxy inférieur, phényle ou benzyle; $A_4$ est un atome d'hydrogène ou un groupe méthyle ou méthoxy; $A_5$ est un atome d'hydrogène ou un groupe méthyle ou méthoxy; $A_6$ est un groupe phényle ou un groupe phényle substitué par un atome de fluor, de chlore ou de brome ou bien par un groupe méthyle, méthoxy ou nitro; et $A_7$ est un atome d'hydrogène ou un groupe phényle, ou bien un groupe phényle substitué par un atome de fluor, de chlore ou de brome ou par un groupe méthyle, méthoxy ou nitro.

9. Composé suivant la revendication 1, caractérisé en ce que la fraction molaire $CHR_3R_4$ dans les composés de formule (I)—(IV) est l'isomère R ou l'isomère S.

10. 9-O-2-(2-Benzyloxycarbonyl)acétamido-éthyl-clavulanate de benzyle.

11. 9-O-2-(2-Carboxy)acétamido-éthyl-clavulanate disodique.

12. 9-O-[N-(N'-Benzyloxycarbonyl-L-alanyl)-2-aminoéthyl]clavulanate de benzyle.

13. Acide 9-O-[N-L-alanyl]-2-aminoéthyl-clavulanique.

14. 9-O-[N-(N'-Benzyloxycarbonyl-glycyl)-2-aminoéthyl]clavulanate de benzyle.

15. Acide 9-O-(N-glycyl)-2-aminoéthyl-clavulanique.

16. 9-O-[2-(3-Benzyloxycarbonyl)propionamido]éthyl-clavulanate de benzyle.

17. 9-O-[2-(3-Carboxy)propionamido]éthyl-clavulanate disodique.

18. Acide 9-O-(N-L-prolyl)-2-aminoéthyl-clavulanique.

19. 9-O-[N-(N'-Benzyloxycarbonyl-D-alanyl)-2-aminoéthyl]clavulanate de benzyle.

20. Acide 9-O-(N-D-alanyl)-2-aminoéthyl-clavulanique.

21. 9-O-[2-(3-Benzyloxycarbonyl-2-benzyloxy-carbonylamino)propionamido]éthyl-clavulanate de benzyle.

22. 9-O-(N-DL-$\alpha$-Aspartyl)-2-aminoéthyl-clavulanate de sodium.

23. 9-O-[N-(6-Benzyloxycarbonyl-aminohexanoyl)-2-aminoéthyl]clavulanate de benzyle.

24. 9-O-[N-(6-Aminohexanoyl)-2-aminoéthyl] clavulanate.

25. 9-O-[N-(N'-Benzyloxycarbonyl-$\beta$-alanyl)-2-aminoéthyl]clavulanate de benzyle.

26. Acide 9-O-(N-$\beta$-alanyl)-2-aminoéthyl-clavulanique.

27. 9-O-[N'-(N-benzyloxycarbonyl-phénylalanyl]-2-aminoéthyl-clavulanate de benzyle.

28. 9-O-(N-L-Phénylalanyl)-2-aminoéthyl-clavulanate.

29. 9-O-[N-L-$\beta$(N'-Benzyloxycarbonyl-$\alpha$-benzyl-aspartyl]-2-aminoéthyl-clavulanate de benzyle.

30. 9-O-(N-L-$\beta$-Aspartyl)-2-aminoéthyl-clavulanate de sodium.

31. 9-O-N-(N'-Benzyloxycarbonyl-L-prolyl)-2-aminoéthyl-clavulanate de benzyle.

32. Procédé pour la préparation d'un composé de formule (I) suivant la revendication 1 ou d'un sel ou ester pharmaceutiquement acceptable de celui-ci, caractérisé en ce qu'on élimine la protection d'un composé de formule (V):—

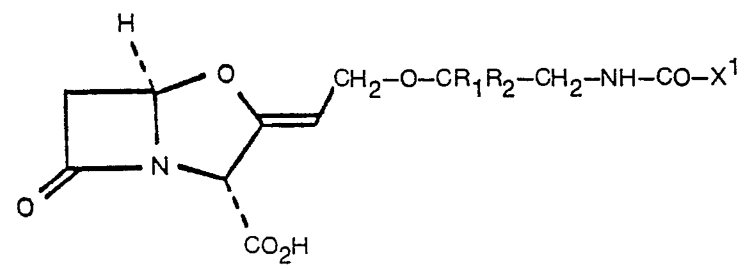

(V)

ou d'un sel ou ester de celui-ci, formule dans laquelle $R_1$, $R_2$ et $R_3$ ont la même signification que dans la revendication 1 et $X^1$ est un groupe de sous-formule (a), (b), (c) ou (d) dans lequel le groupe amino ou le groupe carboxylique est protégé.

33. Procédé pour la préparation d'un composé de formule (V) suivant la revendication 10 ou d'un sel ou ester de celui-ci, caractérisé en ce qu'on effectue la réaction d'un composé de formula (VI):—

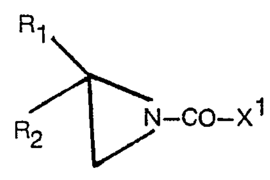

(VI)

dans laquelle $R_1$, $R_2$, $R_3$ et $X^1$ ont la même signification que dans le cas de la formule (V) avec un ester d'acide clavulanique, puis si désiré on effectue ensuite la désestérification de l'ester résultant du composé de formule (V) pour donner le composé de formule (V) ou son sel.

34. Composition pharmaceutique renfermant un composé suivant l'une quelconque des revendications 1 à 31, conjointement à un excipient pharmaceutiquement acceptable.

35. Composition pharmaceutique suivant la revendication 34, renfermant également une

pénicilline ou une céphalosporine, le rapport en poids entre le composé de formule (I) ou son sel ou ester pharmaceutiquement acceptable et la pénicilline ou la céphalosporine étant compris entre 1:1 et 1:8.

36. Composition pharmaceutique suivant la revendication 35, renfermant de 25 à 500 mg d'un composé de formule (I) ou de son sel pharmaceutiquement acceptable et de 150 à 1000 ml d'amoxycilline, d'ampicilline ou d'un composé donnant naissance à ces dernières dans le corps.

37. Composition pharmaceutique suivant l'une quelconque des revendications 34 à 36, renfermant de l'ampicilline trihydrate ou de l'amoxycilline trihydrate.